(19) Europäisches Patentamt
European Patent Office
Office européen des brevets

(11) EP 3 865 129 A1

(12) # EUROPEAN PATENT APPLICATION
published in accordance with Art. 153(4) EPC

(43) Date of publication:
**18.08.2021 Bulletin 2021/33**

(21) Application number: **19871061.8**

(22) Date of filing: **09.10.2019**

(51) Int Cl.:
***A61K 31/222*** *(2006.01)* ***A23L 33/10*** *(2016.01)*
***A61P 1/00*** *(2006.01)* ***A61P 13/10*** *(2006.01)*
***A61P 43/00*** *(2006.01)* ***C07C 69/732*** *(2006.01)*
***C07C 69/736*** *(2006.01)*

(86) International application number:
**PCT/JP2019/039851**

(87) International publication number:
**WO 2020/075764 (16.04.2020 Gazette 2020/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.10.2018 JP 2018191980**

(71) Applicant: **Kao Corporation**
**Chuo-ku,**
**Tokyo 103-8210 (JP)**

(72) Inventors:
- **IWASHITA, Masazumi**
**Haga-gun, Tochigi 321-3497 (JP)**
- **KITAMURA, Naoya**
**Haga-gun, Tochigi 321-3497 (JP)**

(74) Representative: **Vossius & Partner**
**Patentanwälte Rechtsanwälte mbB**
**Siebertstraße 3**
**81675 München (DE)**

(54) **TRPV4 ACTIVITY INHIBITOR**

(57) Provided is a compound that inhibits the activity of TRPV4 and is useful for preventing or ameliorating overactive bladder, irritable bowel syndrome, and the like. A TRPV4 activity inhibitor is provided containing a rosmarinic acid derivative selected from the group consisting of the following formulas (Ia) to (Id), or a salt thereof, as an active ingredient.



Printed by Jouve, 75001 PARIS (FR)

## Description

Technical Field of the Invention

The present invention relates to a rosmarinic acid derivative or a salt thereof useful for preventing or ameliorating overactive bladder, irritable bowel syndrome, and the like.

Background of the Invention

TRPV4 (transient receptor potential cation channel subfamily V member 4) is one of proteins that constitute thermosensitive TRP channels. TRPV4 is expressed in a wide variety of tissues such as the kidney, lung, bladder, heart, skin, brain, and gastrointestinal tract and is considered to play a wide variety of physiological roles.

TRPV4 is particularly strongly expressed in renal distal tubular epithelial cells and it is suggested that the osmotic pressure and the flow rate of urine are perceived by the action of TRPV4 (Non Patent Literature 1). It is also reported that TRPV4 is involved in the function of the bladder (Non Patent Literature 2). That is, TRPV4 is present in the bladder epithelial cells inside of the bladder, and when urine is accumulated and bladder epithelial cells are extended, calcium flows into the inside of the cells through TRPV channels constituted by TRPV4 and the like. This influx of calcium causes a release of ATP from the cells and the expansion of the bladder is transmitted to the nerve. It is also reported that the influence of TRPV4 on the micturition interval is larger than that of other proteins that constitute the TRP channel (Non Patent Literature 3).

Irritable bowel syndrome (IBS) is a disease that causes abdominal discomforts such as diarrhea, constipation, abdominal pain, and lower abdominal bloating because of excessive gas, although no structural disease such as inflammation and ulcer is observed even by performing examinations, unlike diseases such as enteritis due to bacterial and viral infections, ulcerative colitis, and colorectal cancer. It is reported that the IBS patients have a large amount of metabolites (5,6-epoxyeicosatrienoic acid; 5,6-EET) of a polyvalent unsaturated fatty acid which is a TRPV4 agonist in the colon, and when a disrupted solution of the colon containing the 5,6-EET is administered to the mouse colon, the motor reaction of the intestine is increased, leading to the overreaction of the intestine, and in this case, inhibition of the expression of TRPV4 leads to inhibition of the overreaction (Non Patent Literature 4).

Therefore, it is expected to prevent or ameliorate overactive bladder and irritable bowel syndrome by blocking the TRPV4 activity.

On the other hand, it is reported that specific rosmarinic acid derivatives represented by the following structural formulas such as a 3'-O-methyl-rosmarinic acid (i), a 4'-O-methyl-rosmarinic acid (ii), or a 3,3'-O-dimethyl-rosmarinic acid (iii) have an inhibition activity of cyclooxygenase 2 induction and an antiinflammatory action (Patent Literature 2).

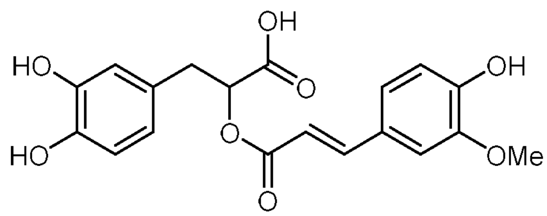

(i)

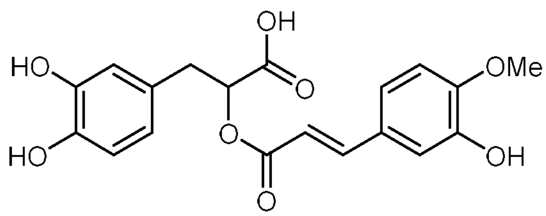

(ii)

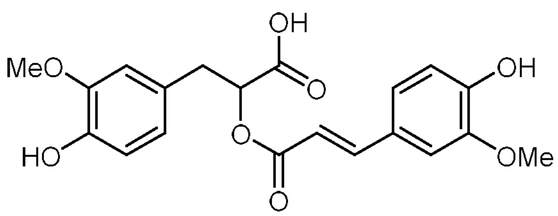

(iii)

[Patent Literature 1] JP-A-2014-24809
[Patent Literature 2] JP-A-2005-272362

[Non Patent Literature 1] Tian W., Am. J. Physiol. Renal. Physiol., 2004, 287, p. F17-F24
[Non Patent Literature 2] Nilius B., Physiol. Rev., 2007, 87, p. 165-217
[Non Patent Literature 3] Gevaert T., J. Clin. Invest., 2007, 117, p. 3453-3462
[Non Patent Literature 4] Nicolas Cenac et al., Gastroenterology 2015 149, 433-444

Summary of the Invention

The present invention relates to the following 1) to 7).

1) A TRPV4 activity inhibitor comprising a rosmarinic acid derivative selected from the group consisting of the following formulas (Ia) to (Id), or a salt thereof, as an active ingredient.
2) An agent for preventing or ameliorating overactive bladder, comprising a rosmarinic acid derivative selected from the group consisting of the following formulas (Ia) to (Id), or a salt thereof, as an active ingredient.
3) An agent for preventing or ameliorating irritable bowel syndrome, comprising a rosmarinic acid derivative selected from the group consisting of the following formulas (Ia) to (Id), or a salt thereof, as an active ingredient.
4) A food for inhibiting TRPV4 activity, comprising a rosmarinic acid derivative selected from the group consisting of the following formulas (Ia) to (Id), or a salt thereof, as an active ingredient.
5) A food for preventing or ameliorating overactive bladder, comprising a rosmarinic acid derivative selected from the group consisting of the following formulas (Ia) to (Id), or a salt thereof, as an active ingredient.
6) A food for preventing or ameliorating irritable bowel syndrome, comprising a rosmarinic acid derivative selected from the group consisting of the following formulas (Ia) to (Id), or a salt thereof, as an active ingredient.
7) A rosmarinic acid derivative represented by the following formula (Ia) or (Ib), or a salt thereof.
8) Use of a rosmarinic acid derivative selected from the group consisting of the following formulas (Ia) to (Id), or a salt thereof, for manufacturing a TRPV4 activity inhibitor.
9) Use of a rosmarinic acid derivative selected from the group consisting of the following formulas (Ia) to (Id), or a salt thereof, for manufacturing an agent for preventing or ameliorating overactive bladder.
10) Use of a rosmarinic acid derivative selected from the group consisting of the following formulas (Ia) to (Id), or a salt thereof, for manufacturing an agent for preventing or ameliorating irritable bowel syndrome.
11) Use of a rosmarinic acid derivative selected from the group consisting of the following formulas (Ia) to (Id), or a salt thereof, for manufacturing a food for inhibiting TRPV4 activity.
12) Use of a rosmarinic acid derivative selected from the group consisting of the following formulas (Ia) to (Id), or a salt thereof, for manufacturing a food for preventing or ameliorating overactive bladder.
13) Use of a rosmarinic acid derivative selected from the group consisting of the following formulas (Ia) to (Id), or a salt thereof, for manufacturing a food for preventing or ameliorating irritable bowel syndrome.
14) A rosmarinic acid derivative selected from the group consisting of the following formulas (Ia) to (Id), or a salt thereof, for use in inhibiting TRPV4 activity.
15) A rosmarinic acid derivative selected from the group consisting of the following formulas (Ia) to (Id), or a salt thereof, for use in preventing or ameliorating overactive bladder.
16) A rosmarinic acid derivative selected from the group consisting of the following formulas (Ia) to (Id), or a salt thereof, for use in preventing or ameliorating irritable bowel syndrome.
17) A non-therapeutic use of a rosmarinic acid derivative selected from the group consisting of the following formulas (Ia) to (Id), or a salt thereof, for inhibiting TRPV4 activity.
18) A non-therapeutic use of a rosmarinic acid derivative selected from the group consisting of the following formulas (Ia) to (Id), or a salt thereof, for preventing or ameliorating overactive bladder.
19) A non-therapeutic use of a rosmarinic acid derivative selected from the group consisting of the following formulas (Ia) to (Id), or a salt thereof, for preventing or ameliorating irritable bowel syndrome.
20) A method for inhibiting TRPV4 activity, the method comprising administering or ingesting an effective amount of a rosmarinic acid derivative selected from the group consisting of the following formulas (Ia) to (Id), or a salt thereof, to a subject in need thereof.
21) A method for preventing or ameliorating overactive bladder, the method comprising administering or ingesting an effective amount of a rosmarinic acid derivative selected from the group consisting of the following formulas (Ia) to (Id), or a salt thereof, to a subject in need thereof.
22) A method for preventing or ameliorating irritable bowel syndrome, the method comprising administering or

ingesting an effective amount of a rosmarinic acid derivative selected from the group consisting of the following formulas (Ia) to (Id), or a salt thereof, to a subject in need thereof.

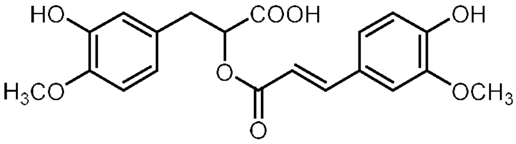

(I a)

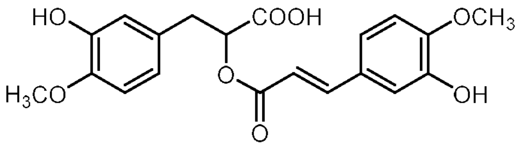

(I b)

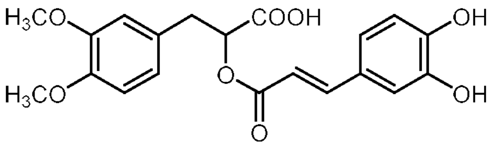

(I c)

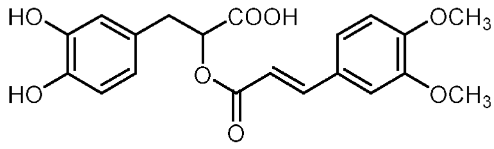

(I d)

Detailed Description of the Invention

The present invention relates to the provision of a compound that inhibits the activity of TRPV4, useful for preventing or ameliorating overactive bladder, irritable bowel syndrome, and the like.

The present inventors conducted studies on rosmarinic acid derivatives and as a result, found that the rosmarinic acid derivatives represented by the above formulas (Ia) to (Id) have an excellent TRPV4 activity inhibiting action and useful for preventing or ameliorating overactive bladder, irritable bowel syndrome, and the like.

The rosmarinic acid derivatives or salts thereof of the present invention can effectively inhibit the TRPV4 activity and are useful for preventing or ameliorating diseases caused by activation of the TRPV4 channel, for example, overactive bladder and irritable bowel syndrome.

The rosmarinic acid derivatives of the present invention are represented by the following formulas (Ia) to (Id), and a compound represented by (Ia) is a 4,3'-di-O-methyl-rosmarinic acid, a compound represented by (Ib) is a 4,4'-di-O-methyl-rosmarinic acid, a compound represented by (Ic) is a 3,4-di-O-methyl-rosmarinic acid, and a compound represented by (Id) is a 3',4'-di-O-methyl-rosmarinic acid. Among them, the compounds represented by (Ia) and (Ib) are new compounds that have not been isolated or synthesized so far.

It should be noted that the compound (Ia) may also be referred to as (E)-3-(3-hydroxy-4-methoxyphenyl)-2-{[3-(4-hydroxy-3-methoxyphenyl)acryloyl]oxy}propionic acid, (Ib) may also be referred to as (E)-3-(3-hydroxy-4-methoxyphenyl)-2-{[3-(3-hydroxy-4-methoxyphenyl)acryloyl]oxy}propionic acid, (Ic) may also be referred to as (E)-3-(3,4-dimethoxyphenyl)-2-{[3-(3,4-dihydroxyphenyl)acryloyl]oxy}propionic acid, and (Id) may also be referred to as (E)-3-(3,4-dihydroxyphenyl)-2-{[3-(3,4-dimethoxyphenyl)acryloyl]oxy}propionic acid.

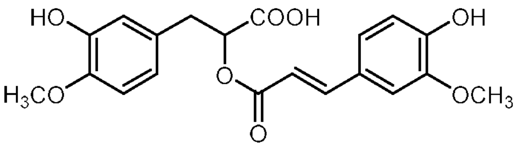

(I a)

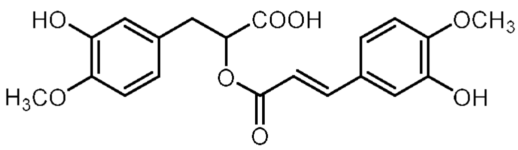

(I b)

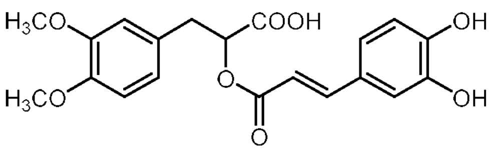

(I c)

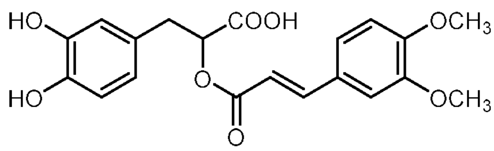

(I d)

There are enantiomers of the rosmarinic acid derivatives of the present invention, and any of the enantiomers or a mixture of the enantiomers may be used. In the present invention, an R enantiomer or an enantiomeric mixture is

preferable.

Examples of the salts of the rosmarinic acid derivatives of the present invention can include salts with alkali metals such as sodium and potassium, salts with alkaline earth metals such as calcium and magnesium, ammonium salts, and salts with amines such as triethylamine.

The rosmarinic acid derivatives of the present invention or salts thereof can be present not only as an unsolvated form, but also as a hydrate or a solvate. Accordingly, the present invention encompasses all crystalline forms and hydrates or solvates thereof. Preferred examples of the solvate include hydrates, alcoholates, and acetone solvates.

The rosmarinic acid derivatives of the present invention can be manufactured by, for example, the methods shown in the Production Examples below. The synthetic schemes of (Ia) to (Id) are illustrated below.

1. Synthetic Example of 4,3'-di-O-methyl-rosmarinic acid (Ia)

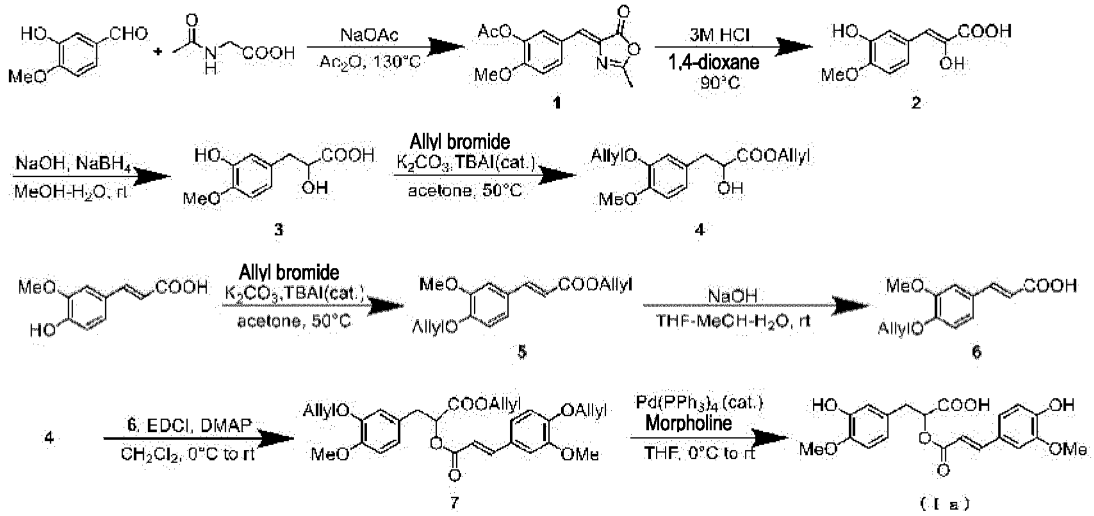


2. Synthetic Example of 4,4'-di-methyl-rosmarinic acid (Ib)

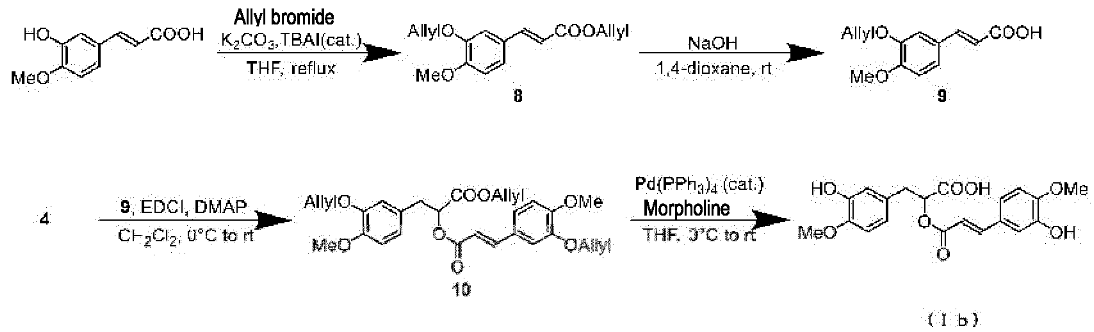


3. Synthetic Example of 3,4-di-O-methyl-rosmarinic acid (Ic)

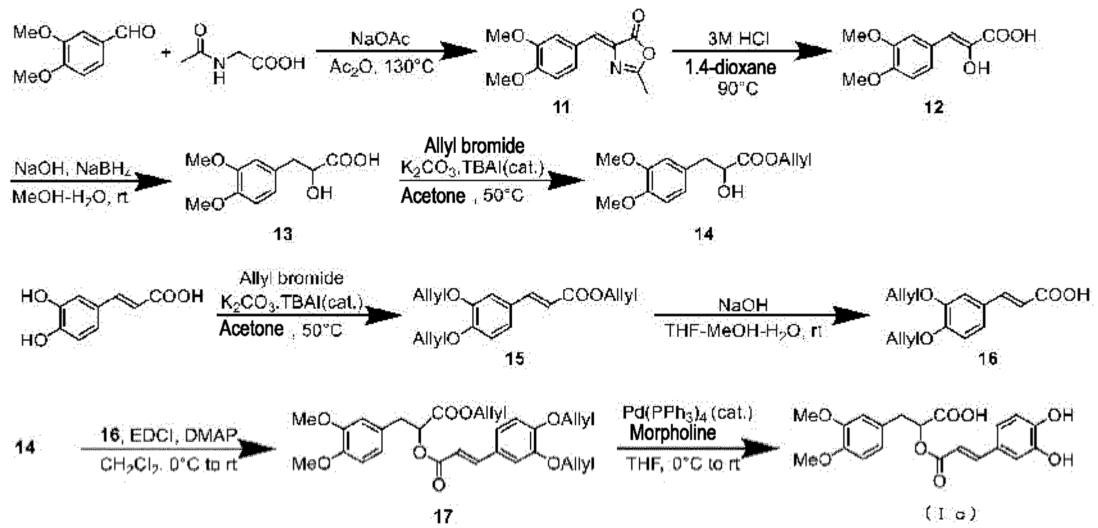


4. Synthetic Example of 3',4'-di-O-methyl-rosmarinic acid (Id)

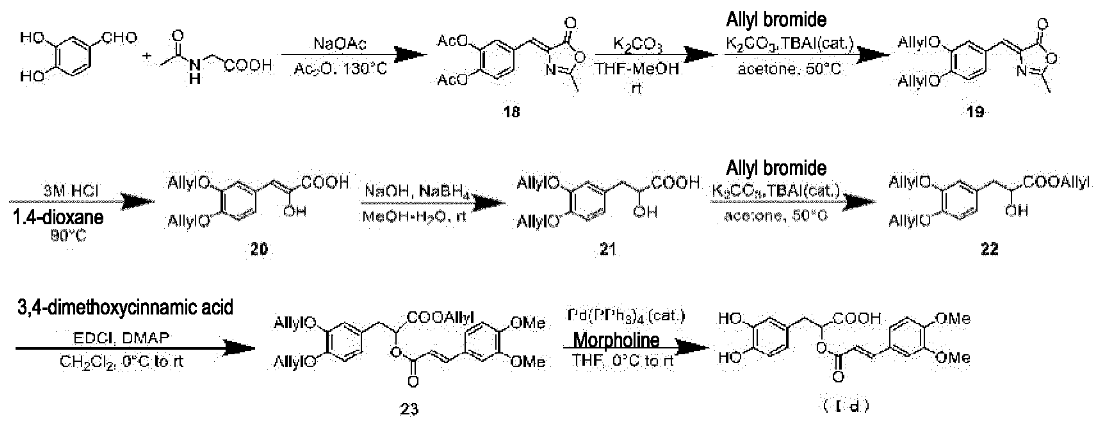


As shown in Examples below, the rosmarinic acid derivatives of the present invention inhibit the TRPV4 activity. That is, the rosmarinic acid derivatives of the present invention has a TRPV4 activity inhibiting action that, when transformed cells in which TRPV4 is functionally expressed (TRPV4 expressing cells) are brought into contact with the rosmarinic acid derivatives by TRPV4 gene introduction in the presence of a TRPV4 stimulant (TRPV4 agonist), the influx of the cation amount in the cells caused by the TRPV4 stimulant is inhibited.

Thus, the rosmarinic acid derivatives of the present invention or salts thereof serve as a TRPV4 activity inhibitor and can be used for inhibiting the TRPV4 activity, or for manufacturing the TRPV4 activity inhibitor.

As mentioned above, TRPV4 is present in the bladder epithelial cells inside of the bladder. When urine is accumulated and bladder epithelial cells are extended, calcium is taken into the inside of the cells through TRPV channels, which causes a release of ATP from the cell surface and the expansion of the bladder is transmitted to the nerve. Therefore, overactive bladder can be prevented or ameliorated by inhibiting the activity of TRPV4. It is reported that irritable bowel syndrome patients have a large amount of 5,6-EET (TRPV4 agonist) which is a metabolite of a polyvalent unsaturated fatty acid, in the colon; administration of a disrupted solution of the colon containing the 5,6-EET to the mouse colon leads to the overreaction of the intestine, and the overreaction is inhibited by inhibiting the expression of TRPV4 (the Non Patent Literature 1), so that irritable bowel syndrome can be prevented or ameliorated by inhibiting the TRPV4 activity.

Thus, the rosmarinic acid derivatives of the present invention or salts thereof serve as an agent for preventing or ameliorating overactive bladder and an agent for preventing or ameliorating irritable bowel syndrome, and can be used for preventing or ameliorating overactive bladder or irritable bowel syndrome, or for manufacturing the agent for preventing or ameliorating overactive bladder or irritable bowel syndrome.

In the present invention, "TRPV4" refers to "transient receptor potential cation channel subfamily V member

4". TRPV4 is a protein encoded by the TRPV4 gene in humans.

The "inhibition of the activity of TRPV4" refers to inhibit the activity of TRPV4 which is a receptor. Specifically, it refers to the inhibition or the blockage of the activity expressed by binding the TRPV4 stimulant to TRPV4, for example, the capacity to regulate ion flux (e.g., capacity to transport cations such as calcium ion and sodium ion from the outside to the inside of cells) and the capacity to regulate membrane potential (e.g., the current generation capacity).

In the present invention, "overactive bladder" refers to a symptom syndrome which essentially has urinary urgency, usually accompanying urinary frequency and urge urinary incontinence. "Irritable bowel syndrome" is a generic term of diseases mainly caused by abnormal motility of the large intestine and secretion function, and it refers to the disease which is diagnosed when no abnormality can be found in examinations, but symptoms of alternating diarrhea and constipation persist, unlike enteritis due to bacterial or viral infections or diseases such as ulcerative colitis and colorectal cancer.

In the present invention, the "use" for inhibiting the TRPV4 activity and for preventing or ameliorating overactive bladder or irritable bowel syndrome may be administration or ingestion in an animal including a human, and it may be either therapeutic use or non-therapeutic use. The "non-therapeutic" is a concept including no medical practices, that is, a concept including no surgical, treatment, or diagnostic process for humans, and more specifically, a concept including no surgical, treatment, or diagnostic process for humans implemented by a physician or a person instructed by a physician.

As used herein, "prevention" refers to prevention or delay of the onset of a disease or a symptom in a subject, or reduction of a risk of the onset of a disease or a symptom in a subject. Additionally, "amelioration" refers to the recovery of a disease, a symptom, or a condition; prevention or delay of the deterioration of a disease, a symptom, or a condition; or reversion, prevention, or delay of the progress of a disease, a symptom, or a condition.

The TRPV4 activity inhibitor and the agent for preventing or ameliorating overactive bladder or irritable bowel syndrome of the present invention may become pharmaceutical products, quasi-pharmaceutical products, supplements, or foods which exert a TRPV4 activity inhibitory effect or an effect of preventing or ameliorating overactive bladder or irritable bowel syndrome when they are ingested or administered to an animal including a human, or may become ingredients or formulations to be incorporated into them.

The food of the present invention encompasses not only general foods and drinks, but also foods which optionally display the concept thereof, functional foods, patient foods, food for specified health uses, foods with function claims, and supplements.

The above pharmaceutical product (including the quasi-pharmaceutical product) containing the rosmarinic acid derivatives of the present invention or salts thereof may be administered in any dosage form, but oral administration is preferable. Upon administration, the pharmaceutical product can be administered in a form of a conventional pharmaceutical formulation by mixing an active ingredient with a solid or liquid non-toxic pharmaceutical carrier which is suitable for an administration method such as an oral administration, an intrarectal administration, or an injection.

Examples of such a formulation include solid agents such as tablets, granules, powders, and capsules, liquid agents such as solutions, suspensions and emulsions, and lyophilization agents. These formulations may be prepared by pharmaceutically common means. If necessary, common additives such as stabilizing agents, moisturizing agents, emulsifying agents, binders, tonicity agents, and excipients can be appropriately added.

Examples of the form of the above foods into which the rosmarinic acid derivatives of the present invention or salts thereof are incorporated include various foods such as foods and drinks and nutritional foods such as soft drinks, tea-based drinks, coffee drinks, fruit juice drinks, carbonated drinks, jelly, wafer, biscuits, breads, noodles, and sausages, and further, nutraceutical compositions having the same form as the above-mentioned oral administration formulations (solid agents such as tablets, capsules, and lozenges). Among them, the tablets are preferable, and chewable tablets are more preferable.

To prepare foods of various forms, the rosmarinic acid derivatives of the present invention or salts thereof may be used singly or may be used by appropriately combining with other food materials, solvents, softener, oils, emulsifying agents, preservatives, flavors, stabilizers, colorants, antioxidants, moisturizing agents, or thickening agents.

The content of the rosmarinic acid derivatives of the present invention or salts thereof in the above pharmaceutical product (including the quasi-pharmaceutical product) or the foods varies depending on the form of use, and is usually preferably 0.01 mass% or more, more preferably 0.1 mass% or more, and further preferably 1 mass% or more, and preferably 100 mass% or less, more preferably 90 mass% or less, and further preferably 70 mass% or less. The content is preferably from 0.01 to 100 mass%, more preferably from 0.1 to 90 mass%, and further preferably from 1 to 70 mass%.

The dosage for a human when the rosmarinic acid derivatives of the present invention or salts thereof are used as a pharmaceutical product or a food, or used by incorporating into a pharmaceutical product or a food varies depending on the conditions, the weight, the sex, the age, or other factors of a subject, and a daily dosage per adult in the case of oral administration is usually, as the rosmarinic acid derivatives or salts thereof, preferably 0.1 mg or more, more preferably 1 mg or more, and further preferably 10 mg or more, and preferably 2,000 mg or less, more preferably 500 mg or less, and further preferably 200 mg or less. It is preferably from 0.1 to 2,000 mg, more preferably from 1 to 500

mg, and further preferably from 10 to 200 mg.

The above formulation may be administered in accordance with any dosage regimen, and it is preferably continuously administered over several weeks to several months, dividing it into once daily or several times per day.

The subject of administration or ingestion is not particularly limited, as long as it is an animal including a human who needs or desires inhibition of the TRPV4 activity or prevention or amelioration of overactive bladder or irritable bowel syndrome, and it is effective to administer or ingest to a human who exhibits symptoms such as dysuria such as overactive bladder or urinary urgency and urge urinary incontinence and a human who exhibits symptoms such as diarrhea, constipation, abdominal pain, and lower abdominal bloating because of excessive gas, caused by stress and the like.

Regarding the above-mentioned embodiments, the present invention discloses the following aspects.

<1> A TRPV4 activity inhibitor comprising a rosmarinic acid derivative selected from the group consisting of the following formulas (Ia) to (Id), or a salt thereof, as an active ingredient.

<2> An agent for preventing or ameliorating overactive bladder, comprising a rosmarinic acid derivative selected from the group consisting of the following formulas (Ia) to (Id), or a salt thereof, as an active ingredient.

<3> An agent for preventing or ameliorating irritable bowel syndrome, comprising a rosmarinic acid derivative selected from the group consisting of the following formulas (Ia) to (Id), or a salt thereof, as an active ingredient.

<4> A food for inhibiting TRPV4 activity, comprising a rosmarinic acid derivative selected from the group consisting of the following formulas (Ia) to (Id), or a salt thereof, as an active ingredient.

<5> A food for preventing or ameliorating overactive bladder, comprising a rosmarinic acid derivative selected from the group consisting of the following formulas (Ia) to (Id), or a salt thereof, as an active ingredient.

<6> A food for preventing or ameliorating irritable bowel syndrome, comprising a rosmarinic acid derivative selected from the group consisting of the following formulas (Ia) to (Id), or a salt thereof, as an active ingredient.

<7> A rosmarinic acid derivative represented by the following formula (Ia) or (Ib), or a salt thereof.

<8> Use of a rosmarinic acid derivative selected from the group consisting of the following formulas (Ia) to (Id), or a salt thereof, for manufacturing a TRPV4 activity inhibitor.

<9> Use of a rosmarinic acid derivative selected from the group consisting of the following formulas (Ia) to (Id), or a salt thereof, for manufacturing an agent for preventing or ameliorating overactive bladder.

<10> Use of a rosmarinic acid derivative selected from the group consisting of the following formulas (Ia) to (Id), or a salt thereof, for manufacturing an agent for preventing or ameliorating irritable bowel syndrome.

<11> Use of a rosmarinic acid derivative selected from the group consisting of the following formulas (Ia) to (Id), or a salt thereof, for manufacturing a food for inhibiting TRPV4 activity.

<12> Use of a rosmarinic acid derivative selected from the group consisting of the following formulas (Ia) to (Id), or a salt thereof, for manufacturing a food for preventing or ameliorating overactive bladder.

<13> Use of a rosmarinic acid derivative selected from the group consisting of the following formulas (Ia) to (Id), or a salt thereof, for manufacturing a food for preventing or ameliorating irritable bowel syndrome.

<14> A rosmarinic acid derivative selected from the group consisting of the following formulas (Ia) to (Id), or a salt thereof, for use in inhibiting TRPV4 activity.

<15> A rosmarinic acid derivative selected from the group consisting of the following formulas (Ia) to (Id), or a salt thereof, for use in preventing or ameliorating overactive bladder.

<16> A rosmarinic acid derivative selected from the group consisting of the following formulas (Ia) to (Id), or a salt thereof, for use in preventing or ameliorating irritable bowel syndrome.

<17> A non-therapeutic use of a rosmarinic acid derivative selected from the group consisting of the following formulas (Ia) to (Id), or a salt thereof, for inhibiting TRPV4 activity.

<18> A non-therapeutic use of a rosmarinic acid derivative selected from the group consisting of the following formulas (Ia) to (Id), or a salt thereof, for preventing or ameliorating overactive bladder.

<19> A non-therapeutic use of a rosmarinic acid derivative selected from the group consisting of the following formulas (Ia) to (Id), or a salt thereof, for preventing or ameliorating irritable bowel syndrome.

<20> A method for inhibiting TRPV4 activity, the method comprising administering or ingesting an effective amount of a rosmarinic acid derivative selected from the group consisting of the following formulas (Ia) to (Id), or a salt thereof, to a subject in need thereof.

<21> A method for preventing or ameliorating overactive bladder, the method comprising administering or ingesting an effective amount of a rosmarinic acid derivative selected from the group consisting of the following formulas (Ia) to (Id), or a salt thereof, to a subject in need thereof.

<22> A method for preventing or ameliorating irritable bowel syndrome, the method comprising administering or ingesting an effective amount of a rosmarinic acid derivative selected from the group consisting of the following formulas (Ia) to (Id), or a salt thereof, to a subject in need thereof.

<23> The content of the active ingredient in the TRPV4 activity inhibitor according to <1> and <8>, the agent for

preventing or ameliorating overactive bladder according to <2> and <9>, the agent for preventing or ameliorating irritable bowel syndrome according to <3> and <10>, the food for inhibiting TRPV4 activity according to <4> and <11>, the food for preventing or ameliorating overactive bladder according to <5> and <12>, and the food for preventing or ameliorating irritable bowel syndrome according to <6> and <13> is preferably 0.01 mass% or more, more preferably 0.1 mass% or more, and further preferably 1 mass% or more, and preferably 100 mass% or less, more preferably 90 mass% or less, and further preferably 70 mass% or less, or preferably from 0.01 to 100 mass%, more preferably from 0.1 to 90 mass%, and further preferably from 1 to 70 mass%, based on the total amount.

<24> A daily dosage per adult in <14> to <23> is, as the rosmarinic acid derivatives represented by the following formulas (Ia) to (Id) or salts thereof, preferably 0.1 mg or more, more preferably 1 mg or more, and further preferably 10 mg or more, and preferably 2,000 mg or less, more preferably 500 mg or less, and further preferably 200 mg or less, or preferably from 0.1 to 2,000 mg, more preferably from 1 to 500 mg, and further preferably from 10 to 200 mg.

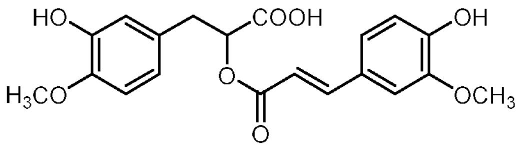

(Ia)

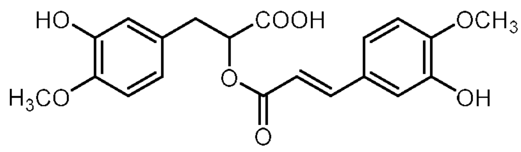

(Ib)

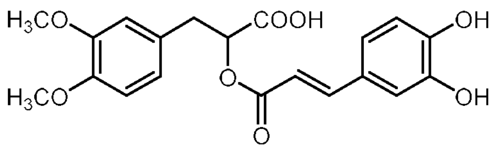

(Ic)

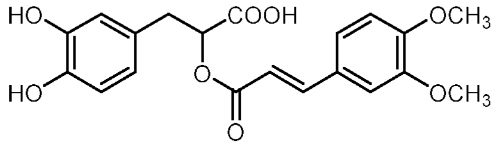

(Id)

Examples

Hereinafter, the present invention will be described further in detail with reference to Examples, but the present invention is not limited thereto.

<Reagent>

Ferulic acid, isoferulic acid, isovanillin, N-acetylglycine, 3,4-dimethoxybenzaldehyde, 3,4-dimethoxycinnamic acid, and coffeic acid were purchased from Tokyo Chemical Industry Co., Ltd. and used. Allyl bromide (Allyl-Br), 4-dimethylaminopyridine (DMAP), tetrabutylammonium iodide (TBAI), tetrakis(triphenylphosphine)palladium (0) complex ($[Pd(PPh_3)_4]$), morpholine, 3,4-dihydroxybenzaldehyde, sodium hydroxide (NaOH), potassium carbonate ($K_2CO_3$), 1,4-dioxane, dichloromethane($CH_2Cl_2$), and tetrahydrofuran (THF) were purchased from FUJIFILM Wako Pure Chemical Corporation and used. 1-ethyl-3-(3-dimethylaminopropyl)carbodiimide hydrochloride (EDCI) was purchased from Dojindo Molecular Technologies, Inc. and used. Sodium acetate (NaOAc) was purchased from Sigma-Aldrich Co. LLC. and used. Acetic anhydride ($Ac_2O$), hydrochloric acid, sodium borohydride ($NaBH_4$), formic acid, methanol (MeOH), acetone, ethyl acetate, hexane, and chloroform ($CHCl_3$) were purchased from Kanto Chemical Co., Inc. and used.

Production Example 1

Synthesis of 4,3'-di-O-methyl-rosmarinic acid (Ia)

1) Synthesis of compound 1

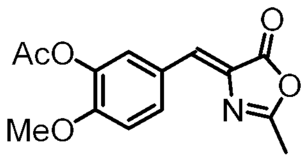

1

After isovanillin (15.22 g, 100 mmol), N-acetylglycine (15.22 g, 130 mmol), and acetic anhydride (50 mL) were added to a 300 mL round-bottom flask, sodium acetate (10.66 g, 130 mmol) was added thereto and stirred for 2 days under heating at 120°C. After cooling to room temperature, the mixture was diluted with ethyl acetate (300 mL) and insoluble materials were filtered using a filter paper. The obtained solution was washed each twice with ion exchange water and saturated saline (200 mL each), dried with anhydrous sodium sulfate, and then, the solvent was distilled off under reduced pressure using an evaporator. The obtained residue was purified by silica gel column chromatography (elution solvent: n-hexane-ethyl acetate (2:1, v/v)) to obtain compound 1 (14.54 g, 52.8 mmol, 53%).

$^{1}$H-NMR (600 MHz, $CDCl_3$) δ 7.81 (d, J = 8.6 Hz, 1H), 7.05 (s, 1H), 7.00 (d, J = 8.6 Hz, 1H), 3.89 (s, 3H), 2.39 (s, 3H), 2.35 (s, 3H). $^{13}$C-NMR (150 MHz, $CDCl_3$) δ 168.84, 167.96, 165.38, 153.67, 139.92, 132.22, 131.20, 130.41, 126.40, 126.17, 112.14, 56.06, 20.70, 15.67.

2) Synthesis of compound 2

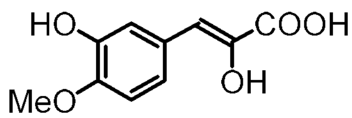

2

After compound 1 (3.85 g, 13.99 mmol) was added to a 300 mL round-bottom flask, 1,4-dioxane (70 mL) and 3 mol/L hydrochloric acid (70 mL) were sequentially added thereto and stirred for 6 hours under heating at 90°C. After cooling to room temperature, the mixture was diluted with ethyl acetate, the obtained organic layer was washed each twice with 1 mol/L hydrochloric acid and saturated saline (200 mL each), dried with anhydrous sodium sulfate, and then, the solvent was distilled off under reduced pressure using an evaporator. The obtained residue was purified by silica gel column chromatography (elution solvent: chloroform-methanol (90:10 → 60:40, v/v)). The obtained product (3.12 g) was used in the next reaction as the crude product.

3) Synthesis of compound 3

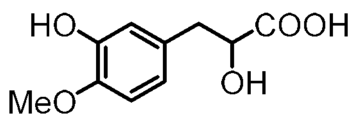

3

Compound 2 (1.58 g, 7.5 mmol) and 25 volume% methanol/ion exchange water (80 mL) were added to a 200 mL round-bottom flask, suspended, and then cooled in an ice bath. Subsequently, 1 mol/L aqueous sodium hydroxide solution (8 mL) was added thereto to obtain a clear solution. At this time, the pH of the solution was 12.0. After adding sodium borohydride (0.43 g, 11.25 mmol), the ice bath was removed, and the mixture was stirred at room temperature for 17 hours. After stirring, the reaction solution was acidified by adding 3 mol/L hydrochloric acid (30 mL) under cooling in an ice bath, thereby stopping the reaction. Subsequently, the reaction solution was extracted three times with ethyl acetate (100 mL), the obtained organic layer was washed with 1 mol/L hydrochloric acid and saturated saline (100 mL each), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (95:5 → 70:30, v/v)). The obtained product (1.05 g) was used in the next reaction as the crude product.

4) Synthesis of compound 4

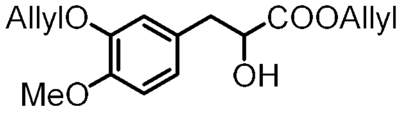

4

After compound 3 (972.2 mg, 4.58 mmol) and tetrabutylammonium iodide (506.0 mg, 1.37 mmol) were added to a 200 mL round-bottom flask, acetone (50 mL)was added thereto and stirred to obtain a clear solution. Subsequently, potassium carbonate (1.90 g, 13.74 mmol) and allyl bromide (1.66 g, 13.74 mmol) were sequentially added thereto and stirred for 16 hours under heating at 50°C. Thereafter, the mixture was cooled to room temperature and diluted with acetone (100 mL), insoluble materials were filtered, and then, the solvent was distilled off under reduced pressure using an evaporator to obtain a reaction residue (1.78 g). The obtained residue was purified by silica gel column chromatography (elution solvent: n-hexane-ethyl acetate (80:20 → 50:50, v/v)) to obtain compound 4 (952.3 mg, 3.26 mmol, 50% (3 steps)).

$^1$H-NMR (600 MHz, $CDCl_3$) δ 6.80 (d, J = 7.9 Hz, 1H), 6.75 (m, 2H), 6.07 (m, 1H), 5.90 (m, 1H), 5.39 (dd, J = 17.2, 1.6 Hz, 1H), 5.34 (dd, J = 17.2, 1.3 Hz, 1H), 5.29 (dd, J = 10.5, 1.2 Hz, 1H), 5.28 (dd, J = 10.6, 1.3 Hz, 1H), 4.65 (m, 2H), 4.59 (m, 2H), 4.44 (dt, J = 6.4, 4.4 Hz, 1H), 3.85 (s, 3H), 3.07 (dd, J = 14.0, 4.5 Hz, 1H), 2.92 (dd, J = 14.1, 6.5 Hz, 1H), 2.72 (d, J = 6.3 Hz, 1H) .$^{13}$C-NMR (150 MHz, $CDCl_3$) δ 173.83, 148.47, 147.74, 133.30, 131.41, 128.50, 122.01, 119.25, 118.03, 114.86, 111.52, 71.32, 69.82, 66.25, 55.94, 40.01.

5) Synthesis of compound 5

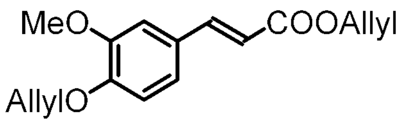

5

After ferulic acid (0.97 g, 5.00 mmol) and tetrabutylammonium iodide (0.59 g, 1.58 mmol) were added to a 200 mL round-bottom flask, acetone (50 mL) was added thereto and stirred to obtain a clear solution. Subsequently, potassium carbonate (2.13 g, 15.4 mmol) and allyl bromide (3.08 g, 25.41 mmol) were added thereto and stirred for 7 hours under heating at 50°C. After stirring, insolubles were removed by suction filtration. The obtained solution was diluted with ethyl acetate (200 mL), washed with 1 mol/L hydrochloric acid and saturated saline (100 mL each), dried with anhydrous sodium sulfate, and then, the solvent was distilled off under reduced pressure using an evaporator. The obtained residue was purified by silica gel column chromatography (elution solvent: n-hexane-ethyl acetate (95:5 → 70:30, v/v)) to obtain compound 5 (1.10 g, 4.01 mmol, 80%).

$^1$H-NMR (600 MHz, $CDCl_3$) δ 7.65 (d, J = 15.9 Hz, 1H), 7.07 (m, 2H), 6.86 (d, J = 8.1 Hz, 1H), 6.34 (d, J = 15.8 Hz, 1H), 6.08 (m, 1H), 6.00 (m, 1H), 5.42 (dd, J = 17.3, 1.4 Hz, 1H), 5.38 (dd, J = 17.3, 1.5 Hz, 1H), 5.31 (dd, J = 10.5, 1.2 Hz, 1H), 5.27 (dd, J = 11.5, 1.4 Hz, 1H), 4.70 (m, 2H), 4.65 (m, 2H), 3.90 (s, 3H).

$^{13}$C-NMR (150 MHz, $CDCl_3$) δ 166.86, 150.12, 149.50, 144.99, 132.73, 132.39, 127.51, 122.47, 118.48, 118.23, 115.57, 112.76, 109.93, 69.73, 65.12, 55.12.

6) Synthesis of compound 6

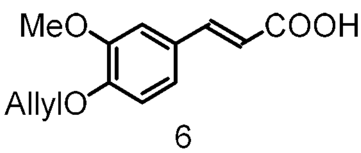

6

Compound 5 (829.5 mg, 3.02 mmol) was charged into a 100 mL round-bottom flask, and then, tetrahydrofuran

(20 mL), methanol (20 mL), and 1 mol/L aqueous sodium hydroxide solution (15 mL) were added thereto and stirred at room temperature for 15 hours. Thereafter, after adding ethyl acetate, the obtained organic layer was washed each twice with 1 mol/L hydrochloric acid and saturated saline (20 mL), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (100:0 → 70:30, v/v)) to obtain compound 6 (678.5 mg, 2.90 mmol, 97%).

$^{1}$H-NMR (600 MHz, DMSO-$d_6$) δ 12.25 (br s, 1H), 7.52 (d, J = 15.9 Hz, 1H), 7.33 (d, J = 1.9 Hz, 1H), 7.18 (dd, J = 8.3, 1.9 Hz, 1H), 6.98 (d, J = 8.4 Hz, 1H), 6.45 (d, J = 16.0 Hz, 1H), 6.04 (m, 1H), 5.40 (dd, J = 17.3, 1.7 Hz, 1H), 5.27 (dd, J = 10.4, 1.6 Hz, 1H), 4.60 (d, J = 5.4 Hz, 1H), 3.82 (s, 3H).

$^{13}$C-NMR (150 MHz, DMSO-$d_6$) δ 168.39, 150.00, 149.60, 144.56, 133.98, 127.72, 122.97, 118.30, 117.30, 113.36, 110.98, 69.26, 56.08.

7) Synthesis of compound 7

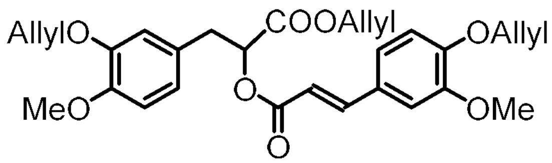

7

Compound 4 (217.2 mg, 0.74 mmol), dichloromethane (10 mL), and 4-dimethylaminopyridine (187.1 mg, 1.52 mmol) were added to a 50 mL round-bottom flask and stirred to obtain a clear solution. Subsequently, compound 6 (234.3 mg, 1.00 mmol) was added and then 1-ethyl-3-(3-dimethylaminopropyl)carbodiimide hydrochloride (287.6 mg, 1.50 mmol) was added while cooling using an ice bath, and then stirred at room temperature for 18 hours. After stirring, the mixture was diluted with ethyl acetate (50 mL), washed with 1 mol/L hydrochloric acid and saturated saline (50 mL each), and dried with anhydrous sodium sulfate, and then, the solvent was distilled off under reduced pressure using an evaporator. The obtained residue was purified by silica gel column chromatography (elution solvent: n-hexane-ethyl acetate (90:10 → 50:50, v/v)) to obtain compound 7 (308.4 mg, 0.606 mmol, 81%).

$^{1}$H-NMR (600 MHz, $CDCl_3$) δ 7.64 (d, J = 15.9 Hz, 1H), 7.06 (m, 2H), 6.86 (d, J = 8.1 Hz, 1H), 6.81 (m, 3H), 6.33 (d, J = 16.0 Hz, 1H), 6.06 (m, 2H), 5.86 (m, 1H), 5.42 (dd, J = 17.2, 1.4 Hz, 1H), 5.34 (m, 4H), 5.25 (dd, J = 10.5, 1.2 Hz, 1H), 5.24 (dd, J = 10.5, 1.2 Hz, 1H), 4.65 (m, 4H), 4.58 (m, 2H), 3.91 (s, 3H), 3.91 (s, 3H), 3.18 (dd, J = 14.5, 4.7 Hz, 1H), 3.13 (dd, J = 14.4, 8.4 Hz, 1H).

$^{13}$C-NMR (150 MHz, $CDCl_3$) δ 169.64, 166.36, 150.34, 149.52, 148.51, 147.76, 146.02, 133.22, 132.67, 131.48, 128.26, 127.31, 122.79, 121.89, 118.83, 118.53, 118.05, 114.71, 114.66, 112.71, 111.55, 109.92, 73.02, 69.88, 69.73, 65.96, 55.95, 55.94, 37.08.

8) Synthesis of 4,3'-di-O-methyl-rosmarinic acid (Ia)

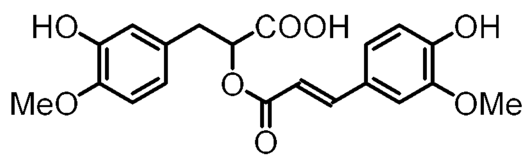

(I a)

Compound 7 (90.2 mg, 0.177 mmol), tetrahydrofuran (5 mL), and morpholine (542.3 mg, 6.23 mmol) were added to a 30 mL round-bottom flask and stirred while cooling using an ice bath to obtain a clear solution. Subsequently, tetrakis(triphenylphosphine)palladium (0) complex (48.5 mg, 0.42 mmol) was added thereto and stirred at room temperature for 24 hours. Thereafter, tetrakis(triphenylphosphine)palladium (0) complex (47.6 mg, 0.41 mmol) was added thereto and stirred at room temperature for 2 hours. Further thereafter, tetrakis(triphenylphosphine)palladium (0) complex (45.8 mg, 0.40 mmol) was added thereto and stirred at room temperature for 24 hours. Subsequently, after the mixture

was diluted with ethyl acetate (50 mL) under an ice bath, the mixture was washed with 1 mol/L hydrochloric acid and saturated saline (30 mL each), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (100:0 → 70:30, v/v)) to obtain 4,3'-di-O-methyl-rosmarinic acid (Ia, 90.2 mg, 0.241 mmol, 57%).

$^1$H-NMR (600 MHz, $CD_3OD$) δ 7.64 (d, J = 15.9 Hz, 1H), 7.11 (d, J = 1.9 Hz, 1H), 7.09 (dd, J = 8.2, 1.9 Hz, 1H), 6.97 (d, J = 8.1 Hz, 1H), 6.83 (d, J = 8.2 Hz, 1H), 6.81 (d, J = 2.1 Hz, 1H), 6.76 (dd, J = 8.2, 2.1 Hz, 1H), 6.38 (d, J = 16.0 Hz, 1H), 5.23 (dd, J = 8.5, 4.2 Hz, 1H), 3.91 (s, 3H), 3.83 (s, 3H), 3.15 (dd, J = 14.4, 4.2 Hz, 1H), 3.06 (dd, J = 14.4, 8.5 Hz, 1H).

Production Example 2

Synthesis of 4,4'-di-O-methyl-rosmarinic acid(Ib)

1) Synthesis of compound 8

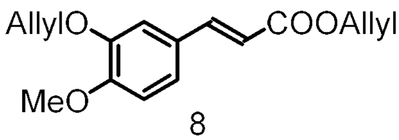

8

Under an argon atmosphere, 7 mL of tetrahydrofuran was added to isoferulic acid (1.50 g, 7.72 mmol). Further, 55% sodium hydride (1.01 g, 23.2 mmol), allyl bromide (16.8 mL, 139 mmol), and TBAI (285 mg, 0.77 mmol) were sequentially added thereto and stirred under the conditions of heating under reflux at 70°C for 24 hours. Thereafter, 16 mL of 8 mol/L aqueous sodium hydroxide solution and 100 mL of ion exchange water were added to the reaction system, which was extracted 3 times with 100 mL of chloroform. After the organic layer was dried with anhydrous sodium sulfate, the solvent was distilled off under reduced pressure. The obtained residue was purified by silica gel column chromatography (elution solvent: n-hexane-ethyl acetate (85:15, v/v)) to obtain 1.88 g (89%) of compound 8.

$^1$H-NMR (600 MHz, $CDCl_3$) δ 7.64 (d, J = 15.9 Hz, 1H), 7.11 (dd, J = 8.3, 2.1 Hz, 1H), 7.06 (d, J = 1.7 Hz, 1H), 6.87 (d, J = 8.5 Hz, 1H), 6.30 (d, J = 15.8 Hz, 1H), 6.09 (ddd, J = 22.6, 10.8, 5.3 Hz, 1H), 6.00 (ddd, J = 22.7, 10.8, 5.5 Hz, 1H), 5.40 (dddd, J = 31.2, 17.1, 2.8, 1.7 Hz, 2H), 5.30 (dddd, J = 27.0, 10.3, 2.5, 1.1 Hz, 2H), 4.71 (dt, J = 5.5, 1.3 Hz, 1H), 4.64 (dt, J = 5.3, 1.4 Hz, 1H), 3.91 (s, 3H).

2) Synthesis of compound 9

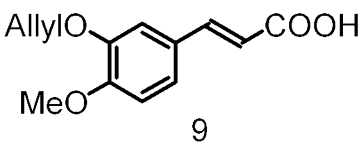

9

Under an argon atmosphere, 34 mL of 1,4-dioxane and 34 mL of 0.4 mol/L aqueous sodium hydroxide solution were added to compound 8 (1.88 g, 6.86 mmol) and stirred at 50°C for 7 hours. Thereafter, 100 mL of ion exchange water was added to the reaction system, which was washed twice with 100 mL of chloroform. Further, 10 mL of 1.5 mol/L aqueous hydrochloric acid solution was added to the aqueous layer, followed by extraction with 100 mL of chloroform three times. After the organic layer was dried with anhydrous sodium sulfate, the solvent was distilled off under reduced pressure to obtain 1.40 g (84%) of compound 9.

$^1$H-NMR (600 MHz, $CDCl_3$) δ 7.71 (d, J = 15.8 Hz, 1H), 7.14 (dd, J = 8.3, 1.9 Hz, 1H), 7.09 (d, J = 1.9 Hz, 1H), 6.89 (d, J = 8.3 Hz, 1H), 6.29 (d, J = 15.8 Hz, 1H), 6.09 (ddd, J = 22.6, 10.5, 5.3 Hz, 1H), 5.44 (ddd, J = 17.3, 3.0, 1.5 Hz, 1H), 5.33 (ddd, J = 10.5, 2.6, 1.1 Hz, 1H), 4.65 (dt, J = 5.7, 1.6 Hz, 2H), 3.92 (s, 3H).

3) Synthesis of compound 10

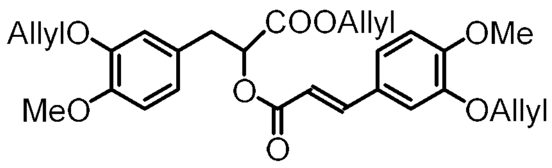

10

Compound 4 (161.9 mg, 0.55 mmol), dichloromethane (10 mL), and 4-dimethylaminopyridine (184.8 mg, 1.50 mmol) were added to a 50 mL round-bottom flask and stirred to obtain a clear solution. Subsequently, compound 9 (234.3 mg, 1.00 mmol) was added and then 1-ethyl-3-(3-dimethylaminopropyl)carbodiimide hydrochloride (287.6 mg, 1.50 mmol) was added thereto while cooling using an ice bath, and then stirred at room temperature for 18 hours. After stirring, the mixture was diluted with ethyl acetate (50 mL), washed with 1 mol/L hydrochloric acid and saturated saline (50 mL each), dried with anhydrous sodium sulfate, and then, the solvent was distilled off under reduced pressure using an evaporator. The obtained residue was purified by silica gel column chromatography (elution solvent: n-hexane-ethyl acetate (90:10 $\rightarrow$ 50:50, v/v)) to obtain compound 10 (282.5 mg, 0.555 mmol, 74%).

$^1$H-NMR (600 MHz, $CDCl_3$) $\delta$ 7.63 (d, J = 15.9 Hz, 1H), 7.10 (dd, J = 8.4, 1.9 Hz, 1H), 7.05 (d, J = 1.9 Hz, 1H), 6.87 (d, J = 8.4 Hz, 1H), 6.81 (m, 3H), 6.30 (d, J = 15.9 Hz, 1H), 6.08 (m, 2H), 5.86 (m, 1H), 5.42 (dd, J = 17.3, 1.5 Hz, 1H), 5.35 (m, 3H), 5.30 (dd, J = 10.7, 1.3 Hz, 1H), 5.25 (dd, J = 10.5, 1.3 Hz, 1H), 5.24 (dd, J = 10.2, 1.2 Hz, 1H), 4.64 (m, 4H), 4.59 (d, J = 5.3 Hz, 1H), 3.91 (s, 3H), 3.85 (s, 3H), 3.18 (dd, J = 14.3, 4.8 Hz, 1H), 3.13 (dd, J = 14.4, 8.4 Hz, 1H).

$^{13}$C-NMR (150 MHz, $CDCl_3$) $\delta$ 169.64, 166.38, 151.78, 148.50, 148.14, 147.76, 146.03, 133.21, 132.83, 131.48, 128.28, 127.01, 123.18, 121.89, 118.82, 118.43, 118.06, 114.71, 114.56, 111.79, 111.55, 111.29, 73.03, 69.87, 65.95, 56.01, 55.94, 37.07.

4) Synthesis of 4,4'-di-O-methyl-rosmarinic acid (Ib)

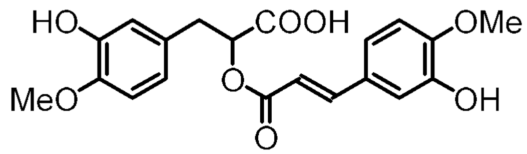

( I b )

Compound 10 (101.7 mg, 0.200 mmol), tetrahydrofuran (5 mL), and morpholine (542.3 mg, 6.23 mmol) were added to a 30 mL round-bottom flask and stirred while cooling using an ice bath to obtain a clear solution. Subsequently, tetrakis(triphenylphosphine)palladium (0) complex (50.1 mg, 0.44 mmol) was added and stirred at room temperature for 24 hours. Thereafter, tetrakis(triphenylphosphine)palladium (0) complex (45.7 mg, 0.40 mmol) was added and stirred at room temperature for 2 hours. Further thereafter, tetrakis(triphenylphosphine)palladium (0) complex(45.8 mg, 0.40 mmol) was added and stirred at room temperature for 24 hours. Subsequently, after the mixture was diluted with ethyl acetate (50 mL) under an ice bath, the mixture was washed with 1 mol/L hydrochloric acid and saturated saline (30 mL each), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (100:0 $\rightarrow$ 70:30, v/v)) to obtain 4,4'-di-O-methyl-rosmarinic acid (compound Ib, 73.9 mg, 0.197 mmol, 99%).

$^1$H-NMR (600 MHz, $CD_3OD$) $\delta$ 7.59 (d, J = 15.9 Hz, 1H), 7.08 (m, 2H), 6.96 (d, J = 8.2 Hz, 1H), 6.87 (d, J = 8.2 Hz, 1H), 6.81 (d, J = 1.9 Hz, 1H), 6.76 (dd, J = 7.9, 2.0 Hz, 1H), 6.33 (d, J = 15.9 Hz, 1H), 5.22 (dd, J = 8.5, 4.4 Hz, 1H), 3.91 (s, 3H), 3.84 (s, 3H), 3.15 (dd, J = 14.3, 4.2 Hz, 1H), 3.05 (dd, J = 14.3, 8.4 Hz, 1H).

Production Example 3

Synthesis of 3,4-di-O-methyl-rosmarinic acid (Ic)

1) Synthesis of compound 11

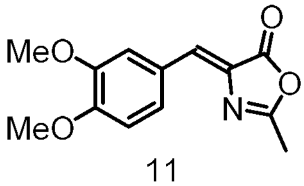

11

After isovanillin (16.62 g, 100 mmol), N-acetylglycine (22.25 g, 190 mmol), and acetic anhydride (70 mL) were added to a 300 mL round-bottom flask, sodium acetate (10.66 g, 130 mmol) was added thereto and stirred for 2 days under heating at 120°C. After cooling to room temperature, the mixture was diluted with ethyl acetate (300 mL) and insoluble materials were filtered using a filter paper. The obtained solution was washed each twice with ion exchange water and saturated saline (200 mL each), dried with anhydrous sodium sulfate, and then, the solvent was distilled off under reduced pressure using an evaporator. The obtained residue was purified by silica gel column chromatography (elution solvent: n-hexane-ethyl acetate (2:1, v/v)) to obtain compound 11 (7.73 g, 31.3 mmol, 31%).

$^1$H-NMR (600 MHz, $CDCl_3$) δ 7.94 (d, J = 2.0 Hz, 1H), 7.51 (dd, J = 8.5, 2.0 Hz, 1H), 7.12 (s, 1H), 6.96 (d, J = 8.3 Hz, 1H), 3.96 (s, 3H), 3.95 (s, 3H), 2.42 (s, 3H).

$^{13}$C-NMR (150 MHz, $CDCl_3$) δ 168.18, 164.90, 151.91, 149.10, 131.70, 130.50, 127.36, 126.44, 113.78, 110.83, 56.01, 55.95, 15.74.2)

Synthesis of compound 13

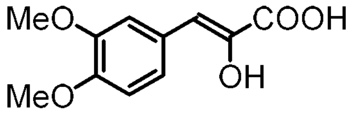

12

After compound 11 (3.92 g, 15.85 mmol) was added to a 300 mL round-bottom flask, 1,4-dioxane(50 mL) and 3 mol/L hydrochloric acid (50 mL) were sequentially added thereto and stirred for 6 hours under heating at 90°C. After cooling to room temperature, the mixture was diluted with ethyl acetate, the obtained organic layer was washed each twice with 1 mol/L hydrochloric acid and saturated saline (200 mL each), dried with anhydrous sodium sulfate, and then, the solvent was distilled off under reduced pressure using an evaporator. The obtained residue was purified by silica gel column chromatography (elution solvent: chloroform-methanol (90:10 → 60:40, v/v)) to obtain compound 12 (3.59 g) quantitatively.

$^1$H-NMR (600 MHz, $CD_3OD$) δ 7.59 (d, J = 1.9 Hz, 1H), 7.28 (dd, J = 8.3, 2.1 Hz, 1H), 6.94 (d, J = 8.4 Hz, 1H), 6.46 (s, 1H), 3.86 (s, 3H), 3.86 (s, 3H).

$^{13}$C-NMR (150 MHz, $CD_3OD$) δ 167.23, 148.61, 148.58, 139.60, 128.37, 122.99, 112.82, 111.06, 110.25, 54.92, 54.90.

3) Synthesis of compound 13

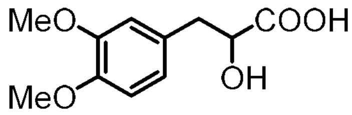

13

Compound 12 (1.57 g, 7.0 mmol) and 25 volume% methanol/ion exchange water (60 mL) was added to a 200 mL round-bottom flask and suspended, and then cooled in an ice bath. Subsequently 1 mol/L aqueous sodium hydroxide solution (6 mL) was added thereto to obtain a clear solution. At this time, the pH of the solution was 12.0. After adding sodium borohydride (0.40 g, 10.50 mmol), the ice bath was removed, and the mixture was stirred at room temperature for 17 hours. After stirring, the reaction solution was acidified by adding 3 mol/L hydrochloric acid (30 mL) under cooling in an ice bath, thereby stopping the reaction. Subsequently, the reaction solution was extracted three times with ethyl acetate (100 mL), the obtained organic layer was washed with 1 mol/L hydrochloric acid and saturated saline (100 mL each), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (95:5 → 70:30, v/v)) to obtain compound 13 (1.10 g, 69%).

$^1$H-NMR (600 MHz, $CD_3OD$) δ 6.90 (d, J = 1.9 Hz, 1H), 6.87 (d, J = 8.2 Hz, 1H), 6.82 (dd, J = 8.2, 2.0 Hz, 1H), 4.33 (dd, J = 7.4, 4.7 Hz, 1H), 3.83 (s, 3H), 3.81 (s, 3H), 3.05 (dd, J = 14.0, 4.3 Hz, 1H), 2.87 (dd, J = 14.0, 7.8 Hz, 1H).

$^{13}$C-NMR (150 MHz, $CD_3OD$) δ 175.77, 148.72, 147.80, 130.30, 121.58, 113.12, 111.46, 71.46, 55.07, 54.93, 39.76.

4) Synthesis of compound 14

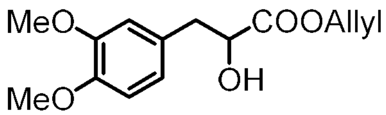

14

After compound 13 (766.5 mg, 3.39 mmol) and tetrabutylammonium iodide (373.1 mg, 1.01 mmol) were added to a 200 mL round-bottom flask, acetone (40 mL) was added thereto and stirred to obtain a clear solution. Subsequently, potassium carbonate (1.40 g, 10.16 mmol) and allyl bromide (2.05 g, 16.94 mmol) were sequentially added thereto and stirred for 16 hours under heating at 50°C. Thereafter, the mixture was cooled to room temperature and diluted with acetone (100 mL), insoluble materials were filtered, and then, the solvent was distilled off under reduced pressure using an evaporator to obtain a reaction residue (1.78 g). The obtained residue was purified by silica gel column chromatography (elution solvent: n-hexane-ethyl acetate (80:20 → 50:50, v/v)) to obtain compound 14 (946.6 mg, 3.55 mmol) quantitatively.

$^1$H-NMR (600 MHz, $CDCl_3$) δ 6.79 (d, J = 8.6 Hz, 1H), 6.76 (m, 2H), 5.91 (m, 1H), 5.36 (dd, J = 17.2 Hz, 1.4 Hz, 1H), 5.29 (dd, J = 10.6 Hz, 1.2 Hz, 1H), 4.66 (m, 2H), 4.45 (m, 1H), 3.86 (s, 3H), 3.85 (s, 3H), 3.09 (dd, J = 14.0 Hz, 4.3 Hz, 1H), 2.94 (dd, J = 14.0 Hz, 6.6 Hz, 1H), 2.75 (d, J = 6.3 Hz, 1H).

$^{13}$C-NMR (150 MHz, $CDCl_3$) δ 173.86, 148.76, 148.00, 131.40, 128.65, 121.58, 119.26, 112.68, 111.08, 71.34, 66.25, 55.87, 55.82, 40.08.

5) Synthesis of compound 15

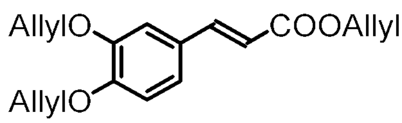

15

After coffeic acid (1.80 g, 10.0 mmol) and tetrabutylammonium iodide (0.74 g, 2.00 mmol) were added to a 200 mL round-bottom flask, acetone (100 mL) was added thereto and stirred to obtain a clear solution. Subsequently,

potassium carbonate (6.91 g, 50.0 mmol) and allyl bromide (6.06 g, 50.0 mmol) were added thereto and stirred for 16 hours under heating at 50°C. After stirring, insolubles were removed by suction filtration. The obtained solution was diluted with ethyl acetate (200 mL), washed with 1 mol/L hydrochloric acid and saturated saline (100 mL each), dried with anhydrous sodium sulfate, and then, the solvent was distilled off under reduced pressure using an evaporator. The obtained residue was purified by silica gel column chromatography (elution solvent: n-hexane-ethyl acetate (95:5 → 70:30, v/v)) to obtain compound 15 (1.77 g, 58.9 mmol, 59%).

$^1$H-NMR (600 MHz, $CDCl_3$) δ 7.63 (d, J = 16.0 Hz, 1H), 7.09 (m, 2H), 6.87 (d, J = 8.8 Hz, 1H), 6.30 (d, J = 16.0 Hz, 1H), 6.07 (m, 2H), 5.98 (m, 1H), 5.43 (m, 2H), 5.37 (dd, J = 17.3, 1.5 Hz, 1H), 5.30 (m, 2H), 5.27 (dd, J = 10.3, 1.2 Hz, 1H), 4.70 (m, 2H), 4.63 (m, 4H).

$^{13}$C-NMR (150 MHz, $CDCl_3$) δ 166.85, 150.62, 148.49, 144.94, 133.04, 132.85, 132.37, 127.46, 122.72, 118.22, 118.03, 117.96, 115.54, 113.30, 112.50, 69.90, 69.69, 65.10.

6) Synthesis of compound 16

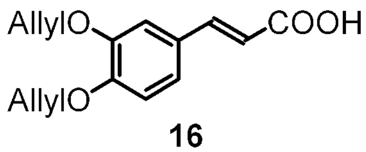

**16**

Compound 15 (1.21 g, 4.03 mmol) was charged to a 100 mL round-bottom flask, and then, tetrahydrofuran (10 mL), methanol (10 mL), and 1 mol/L aqueous sodium hydroxide solution (15 mL) were added thereto and stirred at room temperature for 15 hours. Thereafter, after adding ethyl acetate, the obtained organic layer was washed each twice with 1 mol/L hydrochloric acid and saturated saline (20 mL each), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (100:0 → 70:30, v/v)) to obtain compound 16 (1.10 g) quantitatively.

$^1$H-NMR (600 MHz, DMSO-$d_6$) δ 12.25 (br s, 1H), 7.51 (d, J = 15.9 Hz, 1H), 7.35 (d, J = 1.7 Hz, 1H), 7.20 (dd, J = 8.4, 1.7 Hz, 1H), 7.00 (d, J = 8.4 Hz, 1H), 6.43 (d, J = 15.9 Hz, 1H), 6.05 (m, 2H), 5.42 (m, 2H), 5.27 (m, 2H), 4.62 (m, 4H).

$^{13}$C-NMR (150 MHz, DMSO-$d_6$) δ 168.36, 150.28, 148.40, 144.51, 134.26, 134.01, 127.68, 123.21, 118.00, 117.89, 117.32, 113.74, 112.69, 69.33, 69.21.

7) Synthesis of compound 17

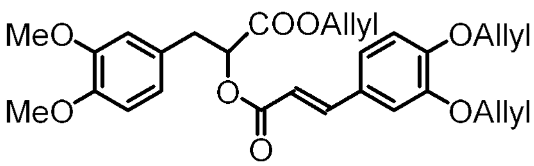

17

Compound 14 (202.1 mg, 0.76 mmol), dichloromethane (10 mL), and 4-dimethylaminopyridine (184.4 mg, 1.50 mmol) were added to a 50 mL round-bottom flask and stirred to obtain a clear solution. Subsequently, compound 16 (234.9 mg, 1.00 mmol) were added thereto and then, 1-ethyl-3-(3-dimethylaminopropyl)carbodiimide hydrochloride (290.9 mg, 1.52 mmol) was added and stirred at room temperature for 18 hours, while cooling using an ice bath. After stirring, the mixture was diluted with ethyl acetate (50 mL), washed with 1 mol/L hydrochloric acid and saturated saline (50 mL each), dried with anhydrous sodium sulfate, and then, the solvent was distilled off under reduced pressure using an evaporator. The obtained residue was purified by silica gel column chromatography (elution solvent: n-hexane-ethyl acetate (90:10 → 50:50, v/v)) to obtain compound 17 (384.4 mg, 0.76 mmol, 100%).

$^1$H-NMR (600 MHz, $CDCl_3$) δ 7.62 (d, J = 15.8 Hz, 1H), 7.06 (m, 2H), 6.86 (d, J = 8.8 Hz, 1H), 6.80 (m, 3H), 6.30 (d, J = 15.9 Hz, 1H), 6.07 (m, 2H), 5.88 (m, 1H), 5.42 (m, 2H), 5.34 (m, 1H), 5.30 (m, 3H), 5.24 (m, 1H), 4.64 (m, 6H), 3.86 (s, 3H), 3.85 (s, 3H), 3.20 (dd, J = 14.3, 4.7 Hz, 1H), 3.15 (dd, J = 14.4, 8.4 Hz, 1H).

$^{13}$C-NMR (150 MHz, $CDCl_3$) δ 169.65, 166.37, 150.87, 148.77, 148.51, 148.05, 146.00, 133.02, 132.82, 131.47,

128.36, 127.27, 123.02, 121.49, 118.84, 118.09, 118.00, 114.64, 113.26, 112.57, 112.48, 111.12, 73.04, 69.95, 69.70, 65.95, 55.87, 55.86, 37.15.

8) Synthesis of 3,4-di-O-methyl-rosmarinic acid (Ic)

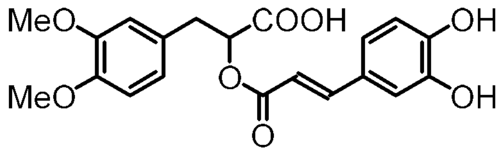

( I c )

Compound 17 (99.9 mg, 0.196 mmol), tetrahydrofuran (5 mL), and morpholine (522.6 mg, 6.00 mmol) were added to a 30 mL round-bottom flask and stirred while cooling using an ice bath to obtain a clear solution. Subsequently, tetrakis(triphenylphosphine)palladium (0) complex (46.8 mg, 0.41 mmol) was added thereto and stirred at room temperature for 24 hours. Thereafter, after the mixture was diluted with ethyl acetate (50 mL) under an ice bath, the mixture was washed with 1 mol/L hydrochloric acid and saturated saline (30 mL each), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (100:0 → 70:30, v/v)) to obtain 3,4-di-O-methyl-rosmarinic acid (compound Ic, 56.5 mg, 0.151 mmol, 77%).

$^1$H-NMR (600 MHz, $CD_3OD$) δ 7.45 (d, J = 15.9 Hz, 1H), 6.93 (d, J = 2.1 Hz, 1H), 6.83 (m, 2H), 6.78 (d, J = 8.2 Hz, 1H), 6.74 (dd, J = 8.1, 2.9 Hz, 1H), 6.68 (d, J = 8.2 Hz, 1H), 6.16 (d, J = 15.9 Hz, 1H), 5.14 (dd, J = 8.5, 4.1 Hz, 1H), 3.72 (s, 3H), 3.70 (s, 3H), 3.10 (dd, J = 14.3, 4.2 Hz, 1H), 3.01 (dd, J = 14.3, 8.7 Hz, 1H).

Production Example 4

Synthesis of 3',4'-di-O-methyl-rosmarinic acid (Id)

1) Synthesis of compound 18

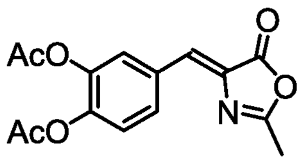

**18**

After 3,4-dihydroxybenzaldehyde (6.91 g, 50 mmol), N- acetylglycine (7.61 g, 65 mmol), and acetic anhydride (50 mL) were added to a 200 mL round-bottom flask, sodium acetate (5.33 g, 65 mmol) was added thereto and stirred for 16 hours under heating at 120°C. After cooling to room temperature, the mixture was diluted with ethyl acetate (300 mL) and insoluble materials were filtered using a filter paper. The obtained solution was washed each twice with ion exchange water and saturated saline (200 mL each), dried with anhydrous sodium sulfate, and then, the solvent was distilled off under reduced pressure using an evaporator. The obtained residue was purified by silica gel column chromatography (elution solvent: n-hexane-ethyl acetate (2:1, v/v)) to obtain compound 18 (9.78 g, 37.4 mmol, 75%).

$^1$H-NMR (600 MHz, $CDCl_3$) δ 8.06 (d, J = 2.0 Hz, 1H), 7.88 (d, J = 8.0, 2.0 Hz, 1H), 7.27 (d, J = 8.0 Hz, 1H), 7.06 (s, 1H), 2.40 (s, 3H), 2.32 (s, 3H), 2.31 (s, 3H).

$^{13}$C-NMR (150 MHz, $CDCl_3$) δ 168.05, 167.80, 167.46, 166.71, 144.16, 142.33, 133.24, 131.84, 130.71, 129.12, 126.70, 123.81, 20.68, 20.62, 15.68.

2) Synthesis of compound 19

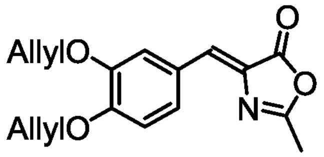

19

Compound 18 (1.42 g, 7.2 mmol), methanol (50 mL), and tetrahydrofuran (50 mL) were added to a 300 mL round-bottom flask and stirred at room temperature to obtain a clear solution. Potassium carbonate (2.90 g, 21.0 mmol) was charged to this solution and stirred for 1 hour. Subsequently, allyl bromide (3.39 g, 28.0 mmol) and tetrabutylammonium iodide (0.77 g, 2.10 mmol) were charged thereto and stirred for 4 hours under heating at 90°C. Thereafter, the obtained solution was diluted with ethyl acetate (200 mL), washed with 1 mol/L hydrochloric acid and saturated saline (100 mL each), dried with anhydrous sodium sulfate, and then, the solvent was distilled off under reduced pressure using an evaporator. The obtained residue was purified by silica gel column chromatography (elution solvent: n-hexane-ethyl acetate (70:30 → 30:70, v/v)) to obtain compound 19 (708.2 mg, 2.37 mmol, 34%).

$^1$H-NMR (600 MHz, $CDCl_3$) δ 7.40 (s, 1H), 7.07 (m, 2H), 6.90 (m, 2H), 6.07 (m, 2H), 5.40 (m, 2H), 5.31 (m, 2H), 4.62 (m, 4H), 2.16 (s, 3H).

3) Synthesis of compound 20

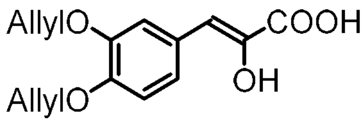

20

After compound 19 (680.0 mg, 2.27 mmol) was added to a 100 mL round-bottom flask, 1,4-dioxane(25 mL) and 3 mol/L hydrochloric acid(25 mL) were sequentially added thereto and stirred for 6 hours under heating at 90°C. After cooling to room temperature, the mixture was diluted with ethyl acetate, the obtained organic layer was washed each twice with 1 mol/L hydrochloric acid and saturated saline (100 mL each), dried with anhydrous sodium sulfate, and then, the solvent was distilled off under reduced pressure using an evaporator. The obtained residue was purified by silica gel column chromatography (elution solvent: 0.1% formic acid/chloroform-methanol (90:10 → 80:20, v/v)) to obtain compound 20 (466.5 mg). The obtained compound 20 was used in the next reaction as the crude product.

4) Synthesis of compound 21

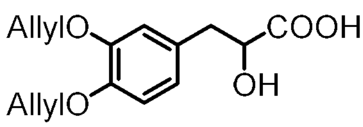

21

Compound 20 (366.5 mg, 1.33 mmol) and 25% methanol/ion exchange water (20 mL) were added to a 100 mL round-bottom flask, suspend, and then cooled in an ice bath. Subsequently 1 mol/L aqueous sodium hydroxide solution (2 mL) was added thereto to obtain a clear solution. At this time, the pH of the solution was 12.0. After sodium borohydride (75.7 mg, 2.00 mmol) was added, the ice bath was removed and the mixture was stirred at room temperature for 17 hours. After stirring, the reaction solution was acidified by adding 3 mol/L hydrochloric acid (10 mL) under cooling in an ice bath, thereby stopping the reaction. Subsequently, the mixture was extracted 3 times with ethyl acetate (30 mL), the obtained organic layer was washed with 1 mol/L hydrochloric acid and saturated saline (30 mL each), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (100:0 → 70:30, v/v)) to obtain compound 24 (250.4 mg). The obtained compound 21 was used in the next reaction as the crude product.

5) Synthesis of compound 22

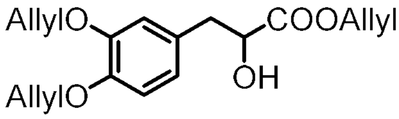

22

After compound 21 (242.4 mg, 0.87 mmol) and tetrabutylammonium iodide (62.8 mg, 0.17 mmol) were added to a 200 mL round-bottom flask, acetone (30 mL) was added thereto and stirred to obtain a clear solution. Subsequently, potassium carbonate (360.7 mg, 2.61 mmol) and allyl bromide (315.8 mg, 2.61 mmol) were sequentially added thereto and stirred for 2 hours under heating at 50°C. Thereafter, the mixture was cooled to room temperature, diluted with ethyl acetate (100 mL), and the obtained organic layer was washed with 1 mol/L hydrochloric acid and saturated saline (30 mL each), dried with anhydrous sodium sulfate, and then, the solvent was distilled off under reduced pressure using an evaporator to obtain a reaction residue (389.5 mg). The obtained residue was purified by silica gel column chromatography (elution solvent: n-hexane-ethyl acetate (80:20 → 50:50, v/v)) to obtain compound 22 (203.6 mg, 37% (3 step)).

$^{1}$H-NMR (600 MHz, $CDCl_3$) δ 6.81 (d, J = 8.2 Hz, 1H), 6.78 (d, J = 1.9 Hz, 1H), 6.73 (dd, J = 8.2, 2.1 Hz, 1H), 6.07 (m, 2H), 5.90 (m, 1H), 5.39 (m, 2H), 5.29 (m, 4H), 4.65 (d, J = 5.8 Hz, 2H), 4.58 (m, 4H), 4.43 (dd, J = 10.8, 6.3 Hz, 1H), 3.05 (dd, J = 14.1, 4.4 Hz, 1H), 2.91 (dd, J = 14.1, 6.7 Hz, 1H), 2.69 (d, J = 6.2 Hz, 1H).

6) Synthesis of compound 23

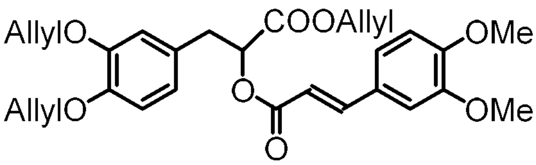

23

Compound 22 (192.4 mg, 0.604 mmol), dichloromethane (15 mL), and 4-dimethylaminopyridine (226.5 mg, 1.85 mmol) were added to a 50 mL round-bottom flask and stirred to obtain a clear solution. Subsequently, after adding 3,4-dimethoxycinnamic acid (249.8 mg, 1.20 mmol), 1-ethyl-3-(3-dimethylaminopropyl)carbodiimide hydrochloride (347.6 mg, 1.81 mmol) was added thereto while cooling using an ice bath, and then stirred at room temperature for 3 hours. After stirring, the mixture was diluted with ethyl acetate (50 mL), washed with 1 mol/L hydrochloric acid and saturated saline (50 mL each), dried with anhydrous sodium sulfate, and then, the solvent was distilled off under reduced pressure using an evaporator. The obtained residue was purified by silica gel column chromatography (elution solvent: n-hexane-ethyl acetate (90:10 → 60:40, v/v)) to obtain compound 23 (288.2 mg, 0.57 mmol, 94%).

$^{1}$H-NMR (600 MHz, $CDCl_3$) δ 7.64 (d, J = 15.8 Hz, 1H), 7.09 (dd, J = 8.3, 1.8 Hz, 1H), 7.04 (d, J = 1.8 Hz, 1H), 6.86 (d, J = 8.3 Hz, 1H), 6.84 (m, 2H), 6.79 (d, J = 8.1 Hz, 1H), 6.33 (d, J = 15.9 Hz, 1H), 6.06 (m, 2H), 5.86 (m, 1H), 5.31 (m, 7H), 4.64 (m, 2H), 4.57 (m, 4H), 3.91 (s, 3H), 3.91 (s, 3H), 3.17 (dd, J = 14.4, 4.7 Hz, 1H), 3.12 (dd, J = 14.4, 8.5 Hz, 1H).

$^{13}$C-NMR (150 MHz, $CDCl_3$) δ 169.61, 166.33, 151.43, 149.29, 148.44, 147.73, 145.99, 133.56, 133.44, 131.51, 128.90, 127.22, 122.96, 122.03, 118.76, 117.56, 117.51, 115.69, 114.69, 114.29, 111.07, 109.72, 72.99, 70.08, 70.05, 65.91, 56.00, 55.94, 37.09.

7) Synthesis of 3',4'-di-O-methyl-rosmarinic acid Id)

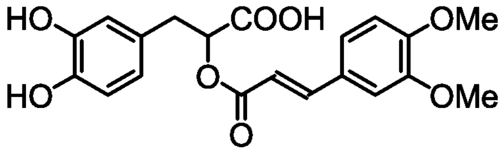

(I d)

Compound 23 (101.7 mg, 0.200 mmol), tetrahydrofuran (5 mL), and morpholine (522.6 mg, 6.00 mmol) were added to a 50 mL round-bottom flask and stirred while cooling using an ice bath to obtain a clear solution. Subsequently, tetrakis(triphenylphosphine)palladium (0) complex (46.6 mg, 0.41 mmol) was added and stirred at room temperature for 24 hours. Thereafter, after the mixture was diluted with ethyl acetate (50 mL) under an ice bath, the mixture was washed with 1 mol/L hydrochloric acid and saturated saline (30 mL each), dried with anhydrous sodium sulfate, and then, the solvent was distilled off under reduced pressure using an evaporator. The obtained residue was purified by silica gel column chromatography (elution solvent: 0.1 volume% formic acid/chloroform-methanol (100:0 → 90:10, v/v)) to obtain 3',4'-di-O-methyl-rosmarinic acid (compound Id, 87.2 mg, 0.233 mmol).

$^1$H-NMR (600 MHz, $CD_3OD$) δ 7.52 (d, J = 15.9 Hz, 1H), 7.11 (d, J = 1.9 Hz, 1H), 7.08 (dd, J = 8.3, 2.0 Hz, 1H), 6.89 (d, J = 8.3 Hz, 1H), 6.67 (d, J = 2.1 Hz, 1H), 6.61 (d, J = 8.1 Hz, 1H), 6.53 (dd, J = 8.0, 2.0 Hz, 1H), 6.31 (d, J = 15.9 Hz, 1H), 5.09 (dd, J = 8.7, 4.1 Hz, 1H), 3.78 (s, 3H), 3.77 (s, 3H), 3.01 (dd, J = 14.4, 4.2 Hz, 1H), 2.91 (dd, J = 14.4, 8.8 Hz, 1H).

Test Example 1

Evaluation of TRPV4 activity inhibiting action

(1) Reagent

The human duodenum-derived cell (Hutu-80 cell) and the human cervical carcinoma-derived cell line (HeLa cell) were purchased from American Type Culture Collection and used; High Pure PCR Product Purification Kit was purchased from Roche and used; pcDNA3.1 Directional TOPO Expression Kit and DMEM/F12 medium were purchased from Invitrogen and used; TransIT-HeLaMONSTER Transfection Kit was purchased from Mirus and used; Detachin was purchased from Genlantis and used; 96well Optical bottom plate was purchased from Nunc and used; Calcium Kit II - fluo 4 was purchased from DOJINDO and used; Hanks' balanced salt solution and fetal bovine serum were purchased from Gibco and used; GSK1016790a was purchased from Sigma Aldrich and used; and 5x PrimeSTAR GXL Buffer, dNTPs mixture, and PrimeSTAR GXL DNA Polymerase were purchased from Takara Bio Inc. and used.

(2) Production of human TRPV4 gene expression vector

Polymerase chain reaction (PCR) was performed under the following conditions using a primer set comprising the oligonucleotides represented by the following base sequences which were synthesized with reference to published human TRPV4 gene sequences using a cDNA obtained by reverse-transcribing a totalRNA extracted from the Hutu-80 cell, as a template.

<Primer set>

Forward primer; 5'-CACCATGGCGGATTCCAGCGAAGGCCC-3' (SEQ ID No: 1)
Reverse primer; 5'-CTAGAGCGGGGCGTCATCAGTCC-3' (SEQ ID No: 2)

<PCR conditions>

a) PCR solution composition

| | |
|---|---|
| cDNA (Template) | 15 μL |
| 5x PrimeSTAR GXL Buffer | 10 μL |

(continued)

| | |
|---|---|
| dNTPs mixture (2.5 mM) | 4 μL |
| PrimeSTAR GXL DNA Polymerase | 1 μL |
| Forward Primer (10 μM) | 1 μL |
| Reverse Primer (10 μM) | 1 μL |
| Water | 18 μL |

b) Temperature and cycle conditions

95°C 2 min ↓
98°C 10 sec, 70°C 2 min, 33 cycles

The obtained PCR product was purified using the High Pure PCR Product Purification Kit. The human TRPV4 gene expression vector was produced using the purified PCR product and the pcDNA3.1 Directional TOPO Expression Kit.

(3) Production of human TRPV4 expressing cells

The HeLa cell was cultured using the DMEM/F12 medium containing 10% of fetal bovine serum. The HeLa cell was seeded in a T-75 cell culture flask at $5 \times 10^5$ cells/Flask. After three days of culture, the human TRPV4 gene expression vector (8 μg) produced in the above (2) was transfected into the cell using the TransIT-HeLaMONSTER Transfection Kit and cultured for 1 day.

The cell was detached using the Detachin and seeded at a density of $1.5 \times 10^4$ cells/90 μL/well in the DMEM/F12 medium containing 10% of fetal bovine serum on the 96well Optical bottom plate and cultured for further 1 day.

(4) Measurement of intracellular calcium ion influx activity

Measurement of the intracellular calcium ion influx activity was performed using the Calcium Kit II - fluo 4. After the Loading buffer containing the Fluo4-AM and the Hanks' balanced salt solution were mixed at a ratio of 1:1, 180 μL/well of the mixture was added to the human TRPV4 expressing cell produced in the above (3) and incubated at 37°C for 1 hour. Thereafter, fluorescence intensity (excitation wavelength: 488 nm, fluorescence wavelength: 524 nm) was measured using a fluorescent plate reader FDSS3000 (Hamamatsu Photonics K.K.) at 37°C every 2 seconds. The GSK1016790a which is the TRPV4 agonist and each of the 4,3'-di-O-methyl-rosmarinic acid, 3',4'-di-O-methyl-rosmarinic acid, 4,4'-di-O-methyl-rosmarinic acid, and 3,4-di-O-methyl-rosmarinic acid solution prepared in Production Examples as samples were diluted with the Hanks' balanced salt solution, 20 μL/well of the mixed solution thereof (they were mixed just before the addition) was added to the cell after 30 seconds after the start of the measurement, and the change of the fluorescence intensity was measured every 2 seconds until 300 seconds. The GSK1016790a was added so as to have a final concentration of 3 nM.

As a positive control of the TRPV4 activity inhibition, HC067047, which is the TRPV4 antagonist, was added to the cell in a final concentration of 10 μM.

The TRPV4 activity of the samples was calculated by the following equation, assuming that the influx rate of calcium ions by the treatment of the GSK1016790a, which is the TRPV4 agonist, is 100%.

(Expression 1)

$$\text{Influx rate of calcium ions (\%)} = [(Fmax/F0) - (FmaxC2/F0C2)]/[(FmaxC1/F0C1) - (FmaxC2/F0C2)] \times 100$$

Fmax: The maximum fluorescence intensity of the well to which the GSK1016790a and a sample were added, at from 0 to 300 seconds after the start of the measurement

FmaxC1: The maximum fluorescence intensity of the well to which the GSK1016790a and a solvent were added, at from 0 to 300 seconds after the start of the measurement

FmaxC2: The maximum fluorescence intensity of the well to which only a solvent was added, at from 0 to 300 seconds after the start of the measurement

F0: The fluorescence intensity of the same well as Fmax, immediately after the start of the measurement
FOCI: The fluorescence intensity of the same well as FmaxC1, immediately after the start of the measurement
FOC2: The fluorescence intensity of the well to which only a solvent was added, immediately after the start of the measurement

The influx rates of calcium ions (n = 3 for each group) in the case of adding samples were tested using the Dunnett's test by comparing with the case of adding the GSK1016790a + a solvent. Table 1 shows the results in the case of adding the 4,3'-di-O-methyl-rosmarinic acid (Ia), the 3',4'-di-O-methyl-rosmarinic acid (Id), the 4,4'-di-O-methyl-rosmarinic acid (Ib), and the 3,4-di-O-methyl-rosmarinic acid (Ic) (in the table, the GSK1016790a, the 4,3'-di-O-methyl-rosmarinic acid, the 3',4'-di-O-methyl-rosmarinic acid, the 4,4'-di-O-methyl-rosmarinic acid, and the 3,4-di-O-methyl-rosmarinic acid are respectively abbreviated as GSK, 4,3'-DiMe-RA, 3',4'-DiMe-RA, 4,4'-DiMe-RA, and 3,4-DiMe-RA). The results were presented as the average value (Mean) ± standard error (S.E.).

[Table 1]

| | **Intracellular $Ca^{2+}$ influx rate (%)** |
|---|---|
| GSK 3nM + DMSO 0.1% | 100.00 ± 1.57 |
| GSK 3nM + HC067047 10 μM **(Positive control)** | 0.63 ± 0.14(P<0.001) |
| GSK 3nM + 4,3'-DiMe-RA 30 μM | 85.31 ± 8.14(P<0.05) |
| GSK 3nM + 4,3'-DiMe-RA 100 μM | 81.02 ± 1,18(P<0.01) |
| GSK 3nM + 3',4'-DiMe-RA 30 μM | 78.81 ± 3.67(P<0.01) |
| GSK 3nM + 3',4'-DiMe-RA 100 μM | 81.74 ± 1.18(P<0.01) |
| GSK 3nM + 4,4'-DiMe-RA 30 μM | 83.19 ± 4.69(P<0.05) |
| GSK 3nM + 4,4'-DiMe-RA 100 μM | 75.87 ± 3.39(P<0.001) |
| GSK 3nM + 3,4-DiMe-RA 30 μM | 80.66 ± 1.58(P<0.01) |
| GSK 3nM + 3,4-DiMe-RA 100 μM | 78.99 ± 3.27(P<0.01) |
| n = 3, Mean ± S.E. | |

Table 1 shows that the 4,3'-di-O-methyl-rosmarinic acid, the 3',4'-di-O-methyl-rosmarinic acid, the 4,4'-di-O-methyl-rosmarinic acid, and the 3,4-di-O-methyl-rosmarinic acid significantly reduced the calcium ion influx caused by the TRPV4 agonist, similar to the TRPV4 antagonist, which is the positive control.

The above results show that the 4,3'-di-O-methyl-rosmarinic acid, the 3',4'-di-O-methyl-rosmarinic acid, the 4,4'-di-O-methyl-rosmarinic acid, and the 3,4-di-O-methyl-rosmarinic acid inhibit the activity of TRPV4. Further, the results show that the 4,3'-di-O-methyl-rosmarinic acid, the 3',4'-di-O-methyl-rosmarinic acid, the 4,4'-di-O-methyl-rosmarinic acid, and the 3,4-di-O-methyl-rosmarinic acid which have an action of inhibiting the TRPV4 activity are effective to prevent or ameliorate irritable bowel syndrome or overactive bladder.

## Claims

1. A TRPV4 activity inhibitor comprising a rosmarinic acid derivative selected from the group consisting of the following formulas (Ia) to (Id), or a salt thereof, as an active ingredient.

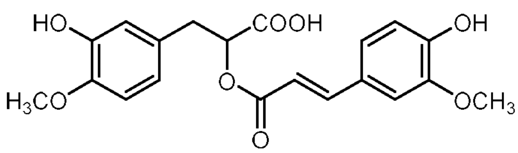

(I a)

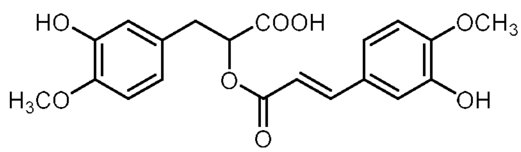

(I b)

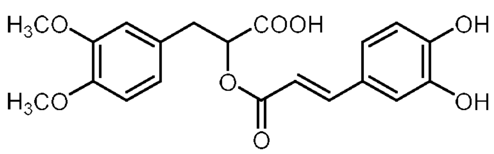

(I c)

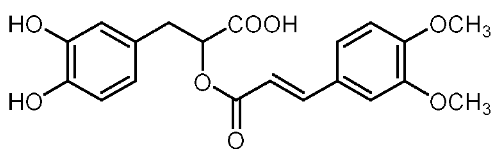

(I d)

2. An agent for preventing or ameliorating overactive bladder, comprising a rosmarinic acid derivative selected from the group consisting of the following formulas (Ia) to (Id), or a salt thereof, as an active ingredient.

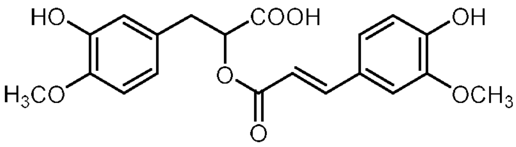
(Ⅰa)

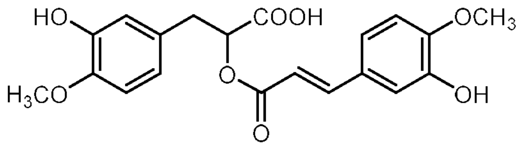
(Ⅰb)

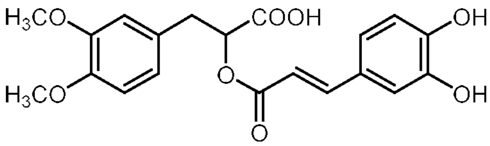
(Ⅰc)

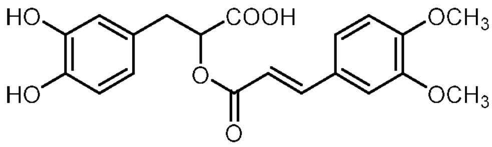
(Ⅰd)

3. An agent for preventing or ameliorating irritable bowel syndrome, comprising a rosmarinic acid derivative selected from the group consisting of the following formulas (Ia) to (Id), or a salt thereof, as an active ingredient.

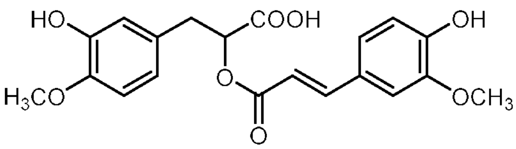
(Ⅰa)

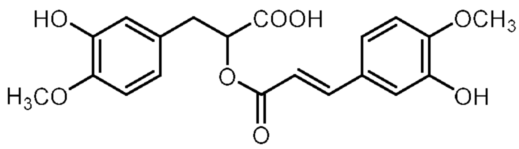
(Ⅰb)

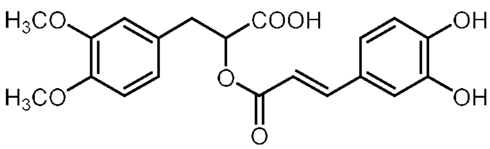
(Ⅰc)

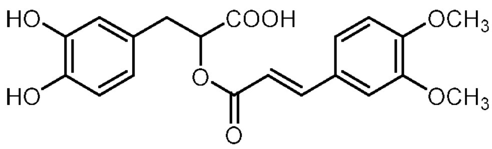
(Ⅰd)

4. A food for inhibiting TRPV4 activity, comprising a rosmarinic acid derivative selected from the group consisting of the following formulas (Ia) to (Id), or a salt thereof, as an active ingredient.

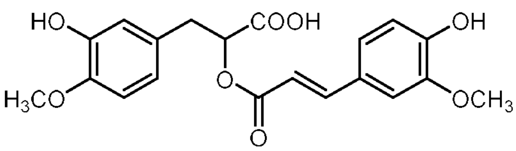
(Ⅰa)

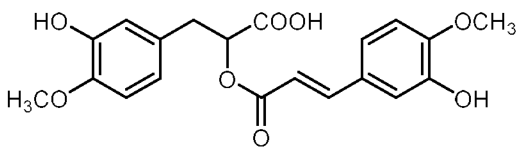
(Ⅰb)

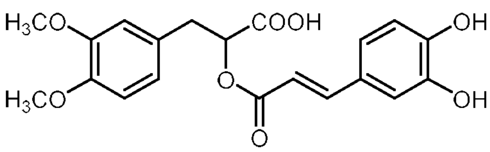
(Ⅰc)

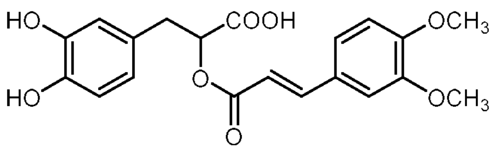
(Ⅰd)

5. A food for preventing or ameliorating overactive bladder, comprising a rosmarinic acid derivative selected from the

group consisting of the following formulas (Ia) to (Id), or a salt thereof, as an active ingredient.

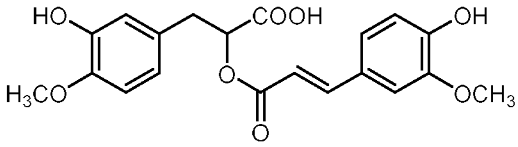

(I a)

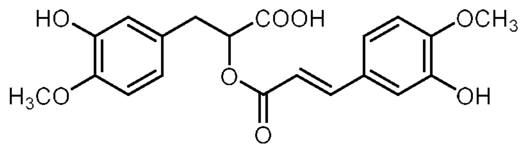

(I b)

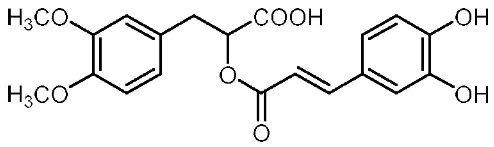

(I c)

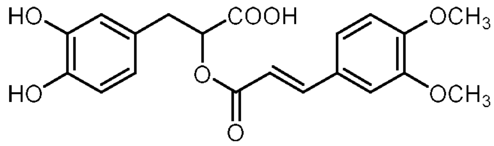

(I d)

6. A food for preventing or ameliorating irritable bowel syndrome, comprising a rosmarinic acid derivative selected from the group consisting of the following formulas (Ia) to (Id), or a salt thereof, as an active ingredient.

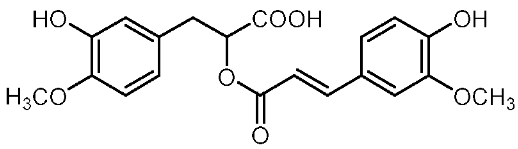

(I a)

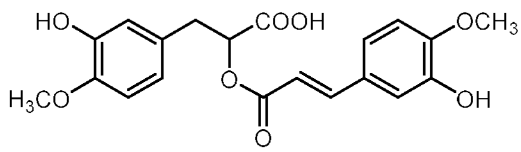

(I b)

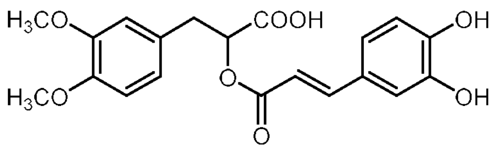

(I c)

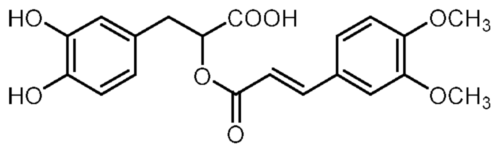

(I d)

7. A rosmarinic acid derivative represented by the following formula (Ia) or (Ib), or a salt thereof.

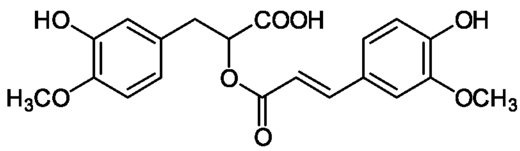

(I a)

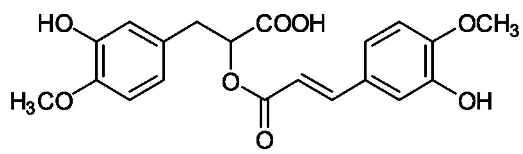

(I b)

8. Use of a rosmarinic acid derivative selected from the group consisting of the following formulas (Ia) to (Id), or a salt thereof, for manufacturing a TRPV4 activity inhibitor.

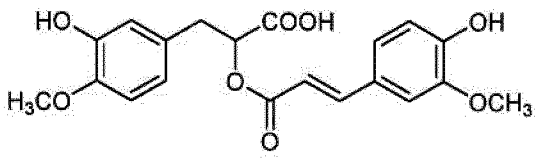

(I a)

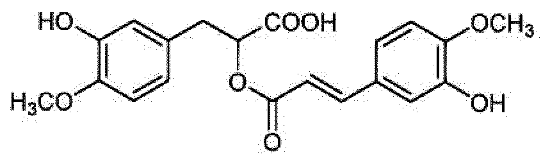

(I b)

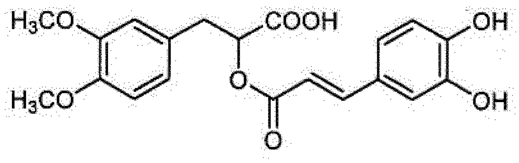

(I c)

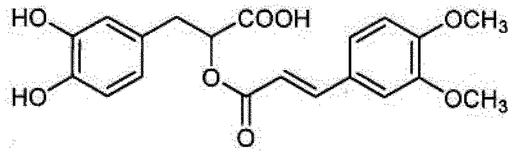

(I d)

**9.** Use of a rosmarinic acid derivative selected from the group consisting of the following formulas (Ia) to (Id), or a salt thereof, for manufacturing an agent for preventing or ameliorating overactive bladder.

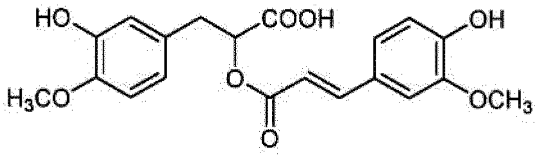

(I a)

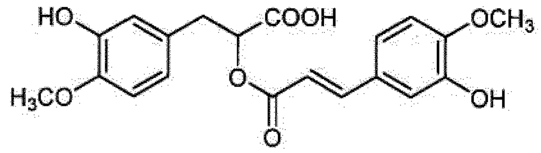

(I b)

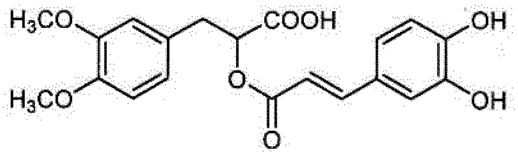

(I c)

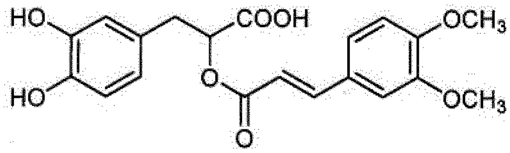

(I d)

**10.** Use of a rosmarinic acid derivative selected from the group consisting of the following formulas (Ia) to (Id), or a salt thereof, for manufacturing an agent for preventing or ameliorating irritable bowel syndrome.

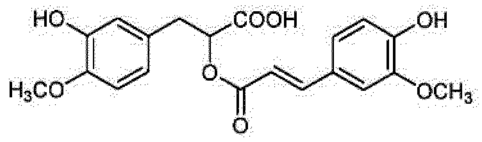

(I a)

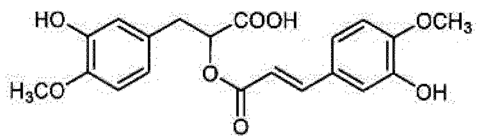

(I b)

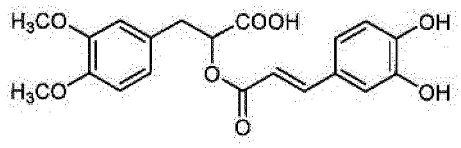

(I c)

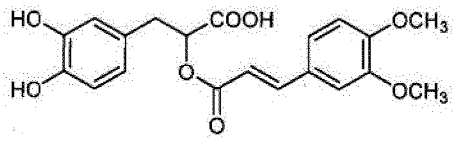

(I d)

**11.** Use of a rosmarinic acid derivative selected from the group consisting of the following formulas (Ia) to (Id), or a salt thereof, for manufacturing a food for inhibiting TRPV4 activity.

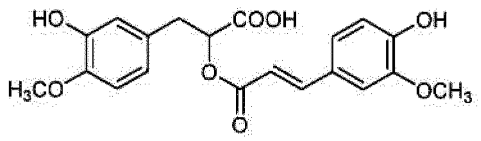

(I a)

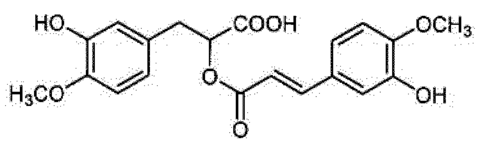

(I b)

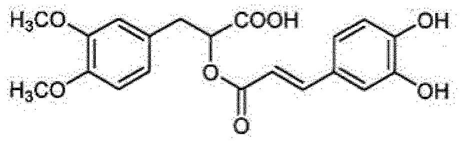
(Ic)

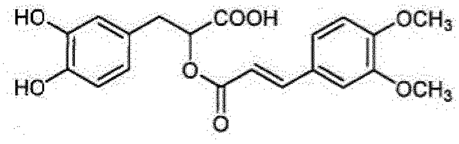
(Id)

**12.** Use of a rosmarinic acid derivative selected from the group consisting of the following formulas (Ia) to (Id), or a salt thereof, for manufacturing a food for preventing or ameliorating overactive bladder.

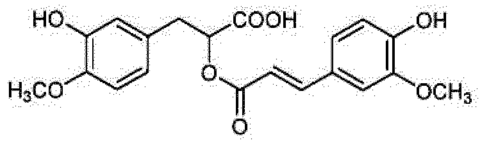
(Ia)

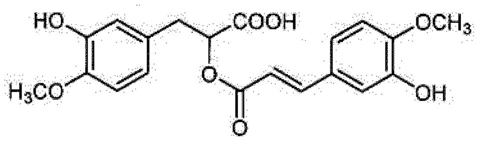
(Ib)

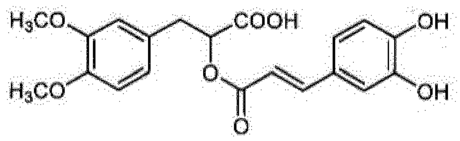
(Ic)

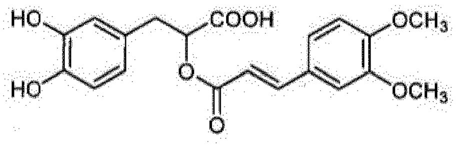
(Id)

**13.** Use of a rosmarinic acid derivative selected from the group consisting of the following formulas (Ia) to (Id), or a salt thereof, for manufacturing a food for preventing or ameliorating irritable bowel syndrome.

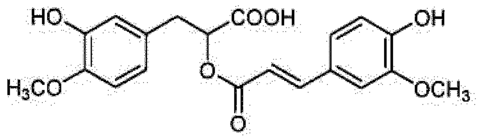
(Ia)

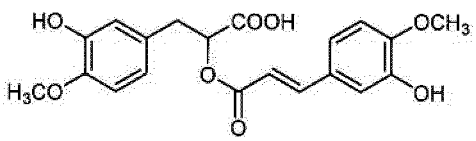
(Ib)

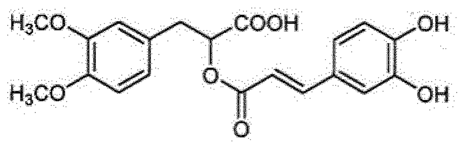
(Ic)

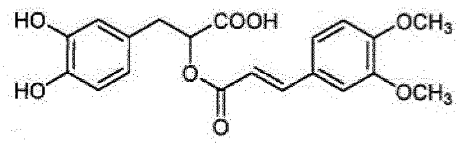
(Id)

**14.** A rosmarinic acid derivative selected from the group consisting of the following formulas (Ia) to (Id), or a salt thereof, for use in inhibiting TRPV4 activity.

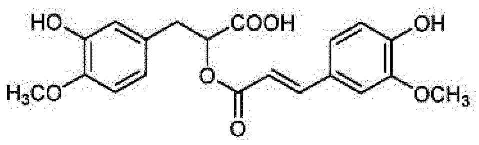
(Ia)

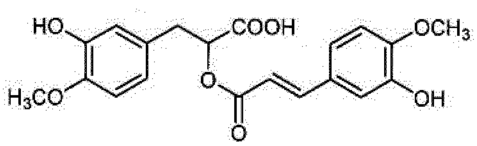
(Ib)

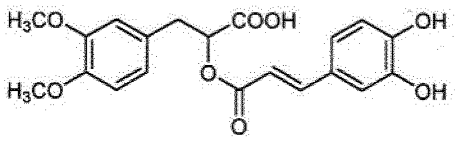

(I c)

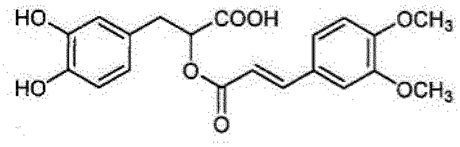

(I d)

**15.** A rosmarinic acid derivative selected from the group consisting of the following formulas (Ia) to (Id), or a salt thereof, for use in preventing or ameliorating overactive bladder.

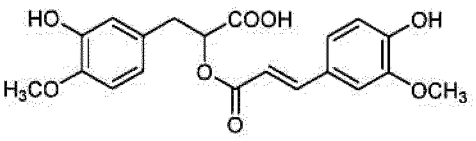

(I a)

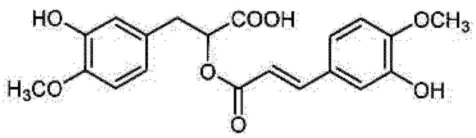

(I b)

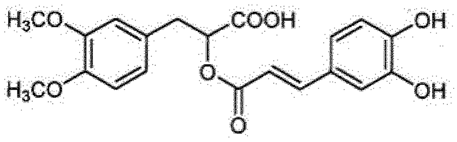

(I c)

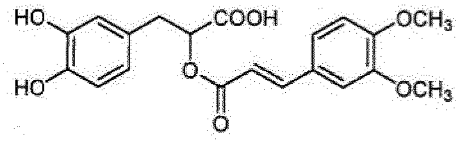

(I d)

**16.** A rosmarinic acid derivative selected from the group consisting of the following formulas (Ia) to (Id), or a salt thereof, for use in preventing or ameliorating irritable bowel syndrome.

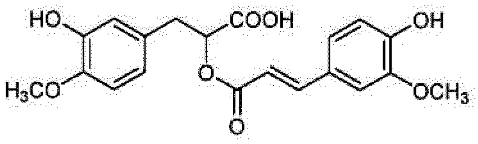

(I a)

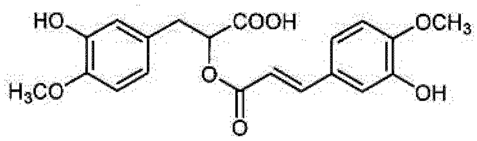

(I b)

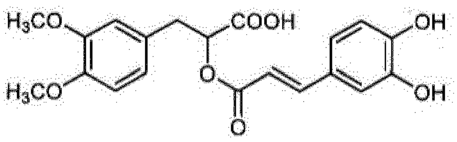

(I c)

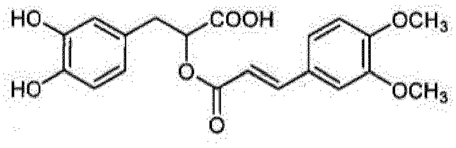

(I d)

**17.** A non-therapeutic use of a rosmarinic acid derivative selected from the group consisting of the following formulas (Ia) to (Id), or a salt thereof, for inhibiting TRPV4 activity.

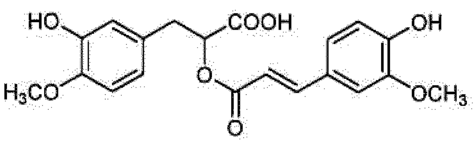

(I a)

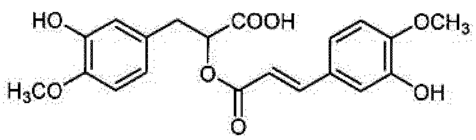

(I b)

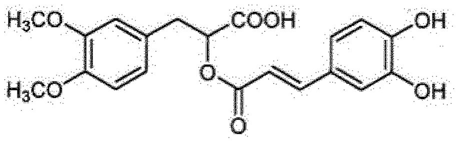

(I c)

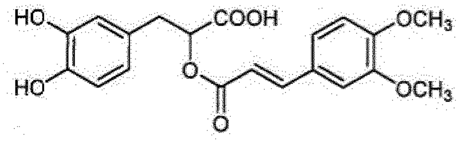

(I d)

**18.** A non-therapeutic use of a rosmarinic acid derivative selected from the group consisting of the following formulas (Ia) to (Id), or a salt thereof, for preventing or ameliorating overactive bladder.

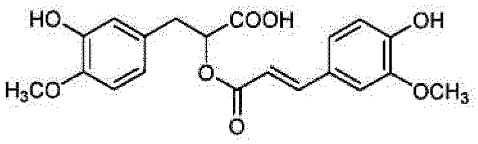

(I a)

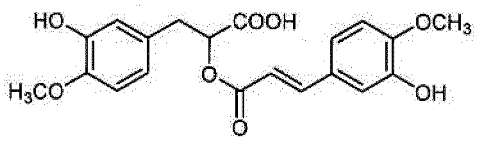

(I b)

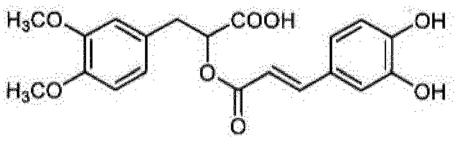

(I c)

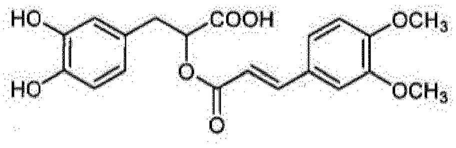

(I d)

**19.** A non-therapeutic use of a rosmarinic acid derivative selected from the group consisting of the following formulas (Ia) to (Id), or a salt thereof, for preventing or ameliorating irritable bowel syndrome.

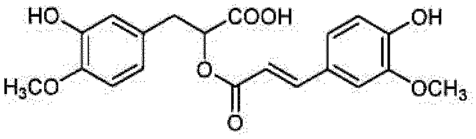

(I a)

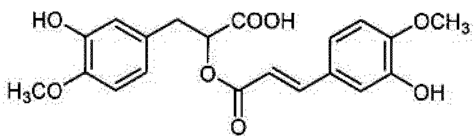

(I b)

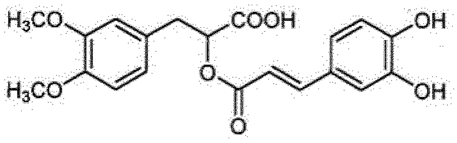

(I c)

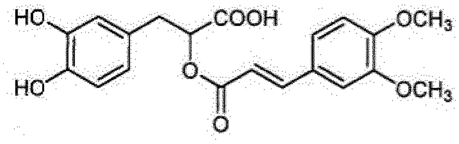

(I d)

**20.** A method for inhibiting TRPV4 activity, the method comprising administering or ingesting an effective amount of a rosmarinic acid derivative selected from the group consisting of the following formulas (Ia) to (Id), or a salt thereof, to a subject in need thereof.

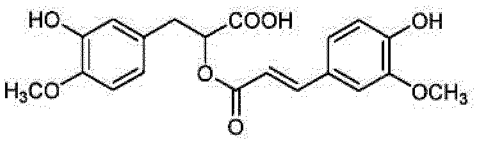

(I a)

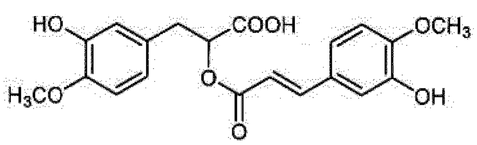

(I b)

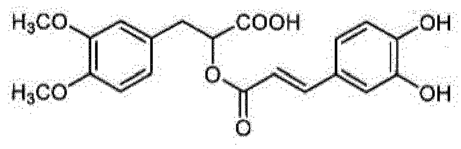

(Ic)

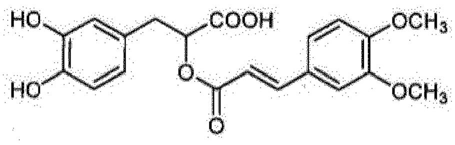

(Id)

**21.** A method for preventing or ameliorating overactive bladder, the method comprising administering or ingesting an effective amount of a rosmarinic acid derivative selected from the group consisting of the following formulas (Ia) to (Id), or a salt thereof, to a subject in need thereof.

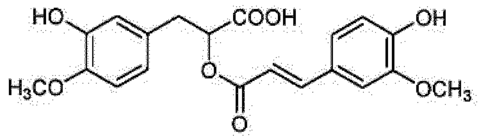

(Ia)

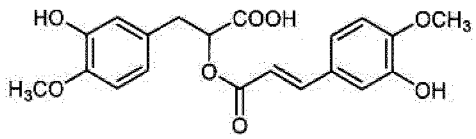

(Ib)

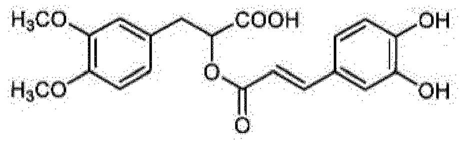

(Ic)

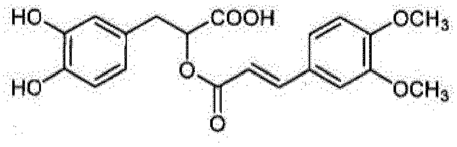

(Id)

**22.** A method for preventing or ameliorating irritable bowel syndrome, the method comprising administering or ingesting an effective amount of a rosmarinic acid derivative selected from the group consisting of the following formulas (Ia) to (Id), or a salt thereof, to a subject in need thereof.

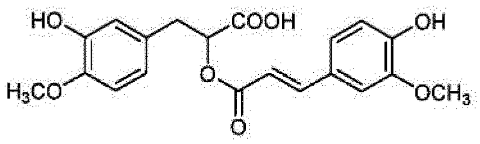

(Ia)

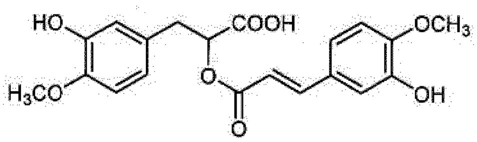

(Ib)

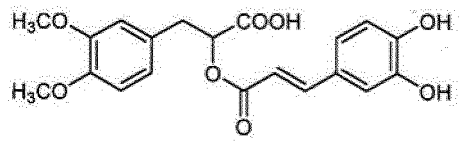

(Ic)

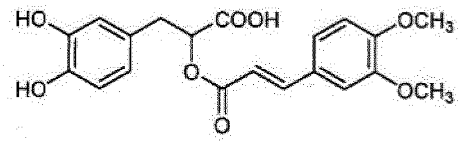

(Id)

## INTERNATIONAL SEARCH REPORT

International application No. PCT/JP2019/039851

A. CLASSIFICATION OF SUBJECT MATTER
Int.Cl. A61K31/222(2006.01)i, A23L33/10(2016.01)i, A61P1/00(2006.01)i, A61P13/10(2006.01)i, A61P43/00(2006.01)i, C07C69/732(2006.01)i, C07C69/736(2006.01)i
According to International Patent Classification (IPC) or to both national classification and IPC

B. FIELDS SEARCHED
Minimum documentation searched (classification system followed by classification symbols)
Int.Cl. A61K31/222, A23L33/10, A61P1/00, A61P13/10, A61P43/00, C07C69/732, C07C69/736

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| P X | JP 2018-177738 A (KAO CORPORATION) 15 November 2018, claims (Family: none) | 1-22 |
| X<br>Y<br><br>A | JP 2005-272362 A (MEIJI SEIKA KAISHA) 06 October 2005, claims (Family: none) | 7, 14-16<br>2, 3, 5, 6, 9, 10, 12, 13, 18, 19, 21, 22<br>1, 4, 8, 11, 17, 20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>07.11.2019 | Date of mailing of the international search report<br>19.11.2019 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2019/039851

C (Continuation). DOCUMENTS CONSIDERED TO BE RELEVANT

| Category* | Citation of document, with indication, where appropriate, of the relevant passages | Relevant to claim No. |
|---|---|---|
| X<br>A | YUAN, H. et al., Synthesis of derivatives of methyl rosmarinate and their inhibitory activities against matrix metalloproteinase-1(MMP-1), European Journal of Medicinal Chemistry, 2013, vol. 62, pp. 148-157, ISSN 0223-5234, abstract, tab. 2, compound 10L | 14-16<br>1-13, 17-22 |
| Y<br><br>A | JP 2005-526040 A (PHARMACIA CORPORATION) 02 September 2005, claims, paragraphs [0005], [0006], [0009], [0090] & US 2003/0191172 A1, claims, paragraphs [0007], [0006], [0010], [0093] & WO 2003/070233 A1 & EP 1915992 A1 & CA 2475374 A & CN 1633283 A & KR 10-2005-0005410 A | 2, 5, 9, 12, 18, 21<br>1, 3, 4, 6-8, 10, 11, 13-17, 19, 20, 22 |
| Y<br><br>A | JP 2003-521511 A (GLAXO GROUP LTD.) 15 July 2003, claims, paragraph [0004] & US 2003/0013717 A1, claims, paragraph [0005] & WO 2001/056555 A2 | 3, 6, 10, 13, 19, 22<br>1, 2, 4, 5, 7-9, 11, 12, 14-18, 20, 21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2014024809 A
- JP 2005272362 A

### Non-patent literature cited in the description

- **TIAN W.** *Am. J. Physiol. Renal. Physiol.,* 2004, vol. 287, F17-F24
- **NILIUS B.** *Physiol. Rev.,* 2007, vol. 87, 165-217
- **GEVAERT T.** *J. Clin. Invest.,* 2007, vol. 117, 3453-3462
- **NICOLAS CENAC et al.** *Gastroenterology,* 2015, vol. 149, 433-444

SEQUENCE LISTING

<110> Kao Corporation

<120> INHIBITOR OF ACTIVATION OF TRPV4

<130> KS1639

<150> JP 2018-191980
<151> 2018-10-10

<160> 2

<170> PatentIn version 3.5

<210> 1
<211> 27
<212> DNA
<213> Artificial Sequence

<220>
<223> Synthetic oligonucleotide

<400> 1
caccatggcg gattccagcg aaggccc 27

<210> 2
<211> 23
<212> DNA
<213> Artificial Sequence

<220>
<223> Synthetic oligonucleotide

<400> 2
ctagagcggg gcgtcatcag tcc 23